# EUROPEAN PATENT APPLICATION

(11) **EP 4 778 944 A1**
(43) Date of publication of application: **22.07.2026**
(21) Application number: 24884964.8
(22) Date of filing: 01.11.2024
(51) Int. Cl.: C07K 19/00, C07K 14/705, A61K 35/17, A61P 35/00

(54) **POLYPEPTIDE CAPABLE OF BINDING TO IMMUNE CHECKPOINT AND USE THEREOF**

(30) Priority: 02.11.2023 CN 202311450930
(71) Applicant: Chongqing Precision Biological Industrial Technology Research Institute Co., Ltd., Chongqing 400039 (CN); Chongqing Precision Biotech Co., Ltd., Chongqing 400038 (CN)
(72) Inventor: ZHAO, Yongchun, Chongqing 400039 (CN); DAI, Depeng, Chongqing 400039 (CN); SHEN, Junjie, Chongqing 400039 (CN); XU, Yanmin, Chongqing 400039 (CN); CHEN, Jun, Chongqing 400039 (CN); QIN, Lei, Chongqing 400039 (CN); HONG, Juan, Chongqing 400039 (CN); ZHAO, Wenxu, Chongqing 400039 (CN); QI, Yanan, Chongqing 400039 (CN); HUANG, Xia, Chongqing 400039 (CN)
(74) Representative: Fabry, Bernd
(86) International application number: PCT/CN2024/129327
(87) International publication number: WO 2025/092967

(57) **Abstract**

Provided is a fusion protein, which comprises a CD226 extracellular region, a transmembrane domain, and a co-stimulatory domain. The fusion protein can significantly enhance the tumor killing ability of a cell expressing an engineered receptor and prolong the survival time of the cell in vivo. Also provided are a nucleic acid molecule comprising a coding sequence of the fusion protein, an engineered cell expressing the fusion protein, and uses of the fusion protein, the nucleic acid molecule, and the engineered cell.

## Description

### TECHNICAL FIELD

The present application mainly relates to the field of cell therapy, and particularly to immune cells expressing a CD226 extracellular segment or a CD226 fusion protein, and a therapy administering the immune cells.

### BACKGROUND

CAR-T cells are T lymphocytes expressing a chimeric antigen receptor, and have achieved breakthrough progress in the treatment of hematological malignancies. However, solid tumors present complex structures, and problems such as tumor heterogeneity and intricate tumor microenvironment hinder CAR-T infiltration and function, while hypoxia and low pH compromise the persistence of CAR-T cells *in vivo.* Studies have shown that in the treatment of solid tumors, improving the persistence of CAR-T cells or enhancing their expansion *in vivo* or at the tumor site can significantly enhance the efficacy of CAR-T in solid tumor therapy.

CD226 is a transmembrane glycoprotein composed of three domains. The first extracellular domain of the CD226 molecule is the structural basis for its ligand recognition, adhesion, immune synapse formation, and cytotoxicity effects. The intracellular region of CD226 contains four tyrosine residues and one serine residue. Known ligands for CD226 include CD155 and CD112. When CD226 binds to its ligands, the CD226 molecule directionally moves to a lipid raft on the cell membrane and recruits intracellular signaling molecules such as PTK and PKC. These molecules are phosphorylated at the four tyrosine residues, thereby activating the cell.

### SUMMARY

The present application primarily relates to application of CD226 in engineered immune effector cells, in particular to application of CD226 in engineered immune effectors. In one aspect, the present application constructs a CD226 fusion protein, wherein the CD226 fusion protein comprises a CD226 extracellular segment, a transmembrane structure, and an intracellular signaling portion. In another aspect, the present application further constructs an engineered immune cell, such as T cell, expressing the CD226 fusion protein and a CAR structure. The engineered cell exhibits enhanced antitumor efficacy against solid tumors and *in vivo* persistence. Herein, targets of the CAR structure include, but are not limited to, solid tumor targets such as CEA, PSCA, CD70, and B7H3. The CD226 fusion protein can enhance the killing ability of CAR-T cells and competes with TIGIT for binding to CD155 and CD112, thereby alleviating immunosuppression in the tumor microenvironment.

Specifically, the present application provides:
1. A fusion protein, from N-terminus to C-terminus, comprising or consisting of:
   1) a CD226 extracellular region, a transmembrane domain, and a co-stimulatory domain; or
   2) a signal peptide, a CD226 extracellular region, a transmembrane domain, and a co-stimulatory domain.

In some embodiments, the signal peptide is a signal peptide derived from a secretory protein.

In some embodiments, the extracellular region and the transmembrane domain are linked via a hinge region. In some embodiments, the hinge region is a hinge region of IgG, IgD, CD8α, or CD28, or a combination thereof.

In some embodiments, the extracellular region and the transmembrane domain are linked via a peptide bond. In some embodiments, the extracellular region and the transmembrane domain, as well as the co-stimulatory domain and the transmembrane domain, are linked directly or indirectly. In some embodiments, the direct linkage is a linkage via covalent or non-covalent bonds. In some embodiments, the indirect linkage is a linkage via a linker. In some embodiments, the linker is a short peptide chain. In some embodiments, the CD226 extracellular region is an extracellular region of human CD226. In some embodiments, the CD226 extracellular region comprises an amino acid sequence as set forth in SEQ ID NO:1 or 3, or a conservative substitution variant of the amino acid sequence as set forth in SEQ ID NO:1 or 3, or an amino acid sequence having at least 85% (e.g., 85%, 86%, 87%, 88%, 89%, 90%, 91%, 92%, 93%, 94%, 95%, 96%, 97%, 98%, 99%, 99.1%, 99.2%, 99.3%, 99.4%, 99.5%, 99.6%, 99.7%, 99.8%, or 99.9% or more) sequence identity to the amino acid sequence as set forth in SEQ ID NO:1 or 3.

2. The fusion protein according to item 1, wherein the transmembrane domain comprises a transmembrane domain of any one or more molecules selected from the following or consists of a transmembrane domain of one or more molecules selected from the following: ICOS, TCRα, TCRβ, TCRγ, TCRδ, CD3ζ, CD3ε, CD3γ, CD3δ, CD4, CD5, CD6, CD7, CD8α, CD9, CD16, CD22, CD27, CD28, CD33, CD37, CD45, CD47, CD52, CD64, CD80, CD86, CD134, 4-1BB, CD152, CD154, CISH, PD-1, and CD226. In some embodiments, the transmembrane domain comprises or is a transmembrane domain of CD28. In some embodiments, the transmembrane domain comprises or is a transmembrane domain of human CD28. In some embodiments, the transmembrane domain comprises or is an amino acid sequence as set forth in SEQ ID NO:17, or a conservative substitution variant of the amino acid sequence as set forth in SEQ ID NO:17, or an amino acid sequence having at least 85% (e.g., 85%, 86%, 87%, 88%, 89%, 90%, 91%, 92%, 93%, 94%, 95%, 96%, 97%, 98%, 99%, 99.1%, 99.2%, 99.3%, 99.4%, 99.5%, 99.6%, 99.7%, 99.8%, or 99.9% or more) sequence identity to the amino acid sequence as set forth in SEQ ID NO:17.

3. The fusion protein according to item 1 or 2, wherein the co-stimulatory domain comprises a signal transduction domain of any one or more molecules selected from the following or consists of a signal transduction domain of one or more molecules selected from the following:
CD27, CD28, 4-1BB, OX40, CD30, CD40, CD2, LFA-1, LIGHT, NKG2C, B7-H3, PD-1, ICOS, CDS, ICAM-1, GITR, BAFFR, LIGHTR, SLAMF7, CD7, NKp80 (KLRF1), CD160, CD19, CD4, CD8α, CD8β, IL2Rβ, IL2Rγ, IL7Rα, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, TNFR2, TRANCE/RANKL, DNAM1, SLAMF4, CD84, CD96, CEACAM1, CRTAM, CD229, CD160, PSGL1, CD100, CD69, SLAMF6, SLAMF1, SLAMF8, CD162, LTBR, LAT, GADS, SLP-76, PAG/Cbp, NKp44, NKp30, NKp46, NKG2D, and a ligand that specifically binds to CD83. In some embodiments, the co-stimulatory domain is a signal transduction domain of CD28. In some embodiments, the co-stimulatory domain is a signal transduction domain of human CD28. In some embodiments, the co-stimulatory domain comprises or is an amino acid sequence as set forth in SEQ ID NO:18, or a conservative substitution variant of the amino acid sequence as set forth in SEQ ID NO:18, or an amino acid sequence having at least 85% (e.g., 85%, 86%, 87%, 88%, 89%, 90%, 91%, 92%, 93%, 94%, 95%, 96%, 97%, 98%, 99%, 99.1%, 99.2%, 99.3%, 99.4%, 99.5%, 99.6%, 99.7%, 99.8%, or 99.9% or more) sequence identity to the amino acid sequence as set forth in SEQ ID NO:18.

4. The fusion protein according to any one of items 1 to 3, wherein the signal peptide comprises or is a signal peptide of CD226 or CD8α molecule. In some embodiments, the signal peptide of the CD8α molecule is a signal peptide of human CD8α molecule, and the signal peptide of the CD226 molecule is a signal peptide of human CD226 molecule. In some embodiments, the signal peptide comprises or is an amino acid sequence as set forth in SEQ ID NO:4, or a conservative substitution variant of the amino acid sequence as set forth in SEQ ID NO:4, or an amino acid sequence having at least 85% (e.g., 85%, 86%, 87%, 88%, 89%, 90%, 91%, 92%, 93%, 94%, 95%, 96%, 97%, 98%, 99%, 99.1%, 99.2%, 99.3%, 99.4%, 99.5%, 99.6%, 99.7%, 99.8%, or 99.9% or more) sequence identity to the amino acid sequence as set forth in SEQ ID NO:4.

5. The fusion protein according to any one of items 1 to 4, from the N-terminus to the C-terminus, comprising or consisting of:
1) the CD226 extracellular region, a CD28 transmembrane domain, and a CD28 signal transduction domain; or
2) a CD226 signal peptide, the CD226 extracellular region, a CD28 transmembrane domain, and a CD28 signal transduction domain.

6. The fusion protein according to any one of items 1 to 5, wherein the CD226 extracellular region comprises or is an amino acid sequence as set forth in SEQ ID NO:1, a conservative substitution variant of the amino acid sequence as set forth in SEQ ID NO:1, or an amino acid sequence having at least 85% (e.g., 85%, 86%, 87%, 88%, 89%, 90%, 91%, 92%, 93%, 94%, 95%, 96%, 97%, 98%, 99%, 99.1%, 99.2%, 99.3%, 99.4%, 99.5%, 99.6%, 99.7%, 99.8%, or 99.9% or more) sequence identity to the amino acid sequence as set forth in SEQ ID NO:1.

7. The fusion protein according to item 6, comprising or consisting of an amino acid sequence as set forth in SEQ ID NO:2, a conservative substitution variant of the amino acid sequence as set forth in SEQ ID NO:2, or an amino acid sequence having at least 85% (e.g., 85%, 86%, 87%, 88%, 89%, 90%, 91%, 92%, 93%, 94%, 95%, 96%, 97%, 98%, 99%, 99.1%, 99.2%, 99.3%, 99.4%, 99.5%, 99.6%, 99.7%, 99.8%, or 99.9% or more) sequence identity to the amino acid sequence as set forth in SEQ ID NO:2.

In some embodiments, the CD226 extracellular region is further directly or indirectly linked to one or more polypeptides or proteins that bind to a tumor antigen or an immune checkpoint protein. In some embodiments, the transmembrane domain is further directly or indirectly linked to one or more polypeptides or proteins that bind to a tumor antigen or an immune checkpoint protein.

8. An engineered receptor, comprising the fusion protein according to any one of items 1 to 6, and a primary signal transduction domain located at the C-terminus of the fusion protein.

9. The engineered receptor according to item 8, wherein the primary signal transduction domain comprises a signal transduction domain of one or more molecules selected from the following or consists of a signal transduction domain of one or more molecules selected from the following: CD3ζ, CD3γ, CD3δ, CD3ε, CD5, CD22, FcRγ, FcRβ, FcεRIγ, FcεRIβ, FcγRIIa, CD79α, CD79β, CD66d, DAP10, and DAP12.

In some embodiments, the primary signal transduction domain is a signal transduction domain portion derived from a human protein that contains an immune-receptor tyrosine-based activation motif.

10. An engineered nucleic acid molecule, encoding the fusion protein according to any one of items 1 to 7 or the engineered receptor according to item 8 or 9. In some embodiments, the engineered nucleic acid molecule is DNA, RNA (e.g., mRNA), or a DNA-RNA hybrid molecule.

In some embodiments, the engineered nucleic acid molecule is chemically modified. For example, in some embodiments, one or more thymidines of the engineered nucleic acid molecule are replaced with uridine. In some embodiments, one or more uridines of the engineered nucleic acid molecule are replaced with thymidine. In some embodiments, one or more guanosines of the engineered nucleic acid molecule are replaced with inosine. In some embodiments, the chemical modification is such that one or more nucleotides of the nucleic acid molecule are replaced with their corresponding nucleotide derivatives. For example, in some embodiments, one or more uridines of the engineered nucleic acid molecule are each replaced with one or more selected from the group consisting of: 5-(carboxyhydroxymethyl)uridine (chm5u), 5-carboxymethylaminomethyluridine (cmnm5u), 5-carboxymethylaminomethyl-2-thiouridine (cmnm5s2u), dihydrouridine (dhu), 2'-O-methylpseudouridine (fm), 1-methylpseudouridine (m1f), 3-(3-amino-3-carboxy-propyl)uridine ((acp3)u), uridine-5-oxyacetic acid (o5u), uridine-5-oxyacetic acid methyl ester (mv), 5-methoxycarbonylmethyluridine (mcm5u), 5-methoxycarbonylmethyl-2-thiouridine (mcm5s2u), 5-methoxyuridine (mo5u), 5-methyl-2-thiouridine (s2t), 2-thiouridine (s2u), 4-thiouridine (s4u), 5-methyluridine (m5u), 2'-O-methyl-5-methyluridine (tm), 2'-O-methyluridine (um), 5-methylaminomethyluridine (mam5u), 5-methylaminomethyl-2-thiouridine (mam5s2u), pseudouridine (p), and 5-methoxycarbonylmethyl-2-thioguanosine (mcm5s2u). In some embodiments, one or more guanosines of the engineered nucleic acid molecule are each replaced with one or more selected from the group consisting of: wybutoxosine (osyw), wybutosine (yw), 1-methylinosine (mli), 2'-O-methylguanosine (gm), 1-methylguanosine (m1g), 2,2-dimethylguanosine (m22g), 2-methylguanosine (m2g), 7-methylguanosine (m7g), β,D-galactosylqueuosine (gal q), queuosine (q), and β,D-mannosylqueuosine (man q). In some embodiments, one or more adenosines of the engineered nucleic acid molecule are each replaced with one or more selected from the group consisting of: N6-isopentenyladenosine (i6a), 1-methyladenosine (m1a), 2-methyladenosine (m2a), N6-methyladenosine (m6a), 2-methylthio-N6-isopentenyladenosine (ms2i6a), N-((9-β-D-ribofuranosyl-2-methylthiopurine-6-yl)carbamoyl)threonine (ms2t6a), N-((9-β-D-ribofuranosylpurin-6-yl)N-methylcarbamoyl)threonine (mt6a), N-((9-β-D-ribofuranosylpurin-6-yl)-carbamoyl)threonine (t6a), queuosine (q), and D-mannosylqueuosine (man q). In some embodiments, one or more cytidines of the engineered nucleic acid molecule are each replaced with one or more selected from the group consisting of: 4-acetylcytidine (ac4c), 2'-O-methylcytidine (cm), 3-methylcytosine (m3c), N4-methylcytidine (m4c), 5-methylcytidine (m5c), β, and 2-thiocytidine (s2c). In some embodiments, the chemical modification includes 2'-O-methylation modification on the ribose of a nucleotide, 3' phosphorothioate bond modification between nucleotides, or both. In some embodiments, the modification is 2'-O-methylation modification on the ribose of the first three nucleotides at the 5' end, 2'-O-methylation modification on the ribose of the last three nucleotides at the 3' end, internucleotide 3' phosphorothioate modification of the first three nucleotides at the 5' end, and internucleotide 3' phosphorothioate modification of the last three nucleotides at the 3' end.

In some embodiments, the nucleic acid molecule further encodes one or more second engineered receptors, wherein the second engineered receptor binds to one or more tumor antigens and/or immune checkpoint proteins. In some embodiments, the second engineered receptor is a chimeric antigen receptor (CAR), a T cell receptor (TCR), or a T cell antigen coupler (TAC). In some embodiments, the one or more immune checkpoint proteins to which the second engineered receptor binds are not CD155. In some embodiments, the one or more immune checkpoint proteins to which the second engineered receptor binds include CD155.

11. An engineered cell, comprising the fusion protein according to any one of items 1 to 7, or the engineered receptor according to item 8 or 9, and/or the engineered nucleic acid molecule according to item 10.

12. The engineered cell according to item 11, further comprising one or more engineered receptors that bind to one or more target molecules, wherein the one or more target molecules are selected from tumor antigens and/or immune checkpoint proteins, wherein
the tumor antigens are selected from the group consisting of: prostate stem cell antigen (PSCA), carcinoembryonic antigen (CEA) CAM5, CD123, thyroid stimulating hormone receptor (TSHR); CD171; CS-1; C-type lectin-like molecule-1; ganglioside GD3; Tn antigen; CD19; CD20; CD22; CD30; CD70; CD123; CD138; CD33; CD44; CD44v7/8; CD38; CD44v6; B7H3 (CD276), B7H6; KIT (CD117); interleukin 13 receptor subunit alpha (IL-13Rα); interleukin 11 receptor alpha (IL-11Rα); prostate specific membrane antigen (PSMA); NY-ESO-1; HIV-1 Gag; MART-1; gp100; tyrosinase; mesothelin; EpCAM; protease serine 21 (PRSS21); vascular endothelial growth factor receptor; Lewis(Y) antigen; CD24; platelet derived growth factor receptor beta (PDGFR-β); stage-specific embryonic antigen-4 (SSEA-4); cell surface associated mucin 1 (MUC1), MUC6; epidermal growth factor receptor family and mutants thereof (EGFR, EGFR2, ERBB3, ERBB4, EGFRvIII); neural cell adhesion molecule (NCAM); carbonic anhydrase IX (CAIX); LMP2; ephrin type-A receptor 2 (EphA2); fucosyl GM1; sialyl Lewis adhesion molecule (sLe); ganglioside GM3 (aNeu5Ac(2-3)bDGalp(1-4)bDGlcp(1-1)Cer); TGS5; high molecular weight melanoma-associated antigen (HMWMAA); O-acetyl-GD2 ganglioside (OAcGD2); folate receptor; tumor endothelial marker 1 (TEM1/CD248); tumor endothelial marker 7-related (TEM7R); Claudin 6, Claudin 18.2, Claudin 18.1; ASGPR1; CDH16; 5T4; 8H9; αvβ6 integrin; B cell maturation antigen (BCMA); CA9; kappa light chain; CSPG4; EGP2, EGP40; FAP; FAR; FBP; embryonic AchR; HLA-A1, HLA-A2; MAGEA1, MAGE3; KDR; MCSP; NKG2D ligand; PSC1; ROR1; Sp17; SURVIVIN; TAG72; TEM1; fibronectin; tenascin; carcinoembryonic variant of tumor necrosis region; G protein-coupled receptor class C group 5 member D (GPRC5D); chromosome X open reading frame 61 (CXORF61); CD97; CD179a; anaplastic lymphoma kinase (ALK); polysialic acid; placenta-specific 1 (PLAC1); hexasaccharide portion of globoH glycoceramide (GloboH); mammary gland differentiation antigen (NY-BR-1); uroplakin 2 (UPK2); hepatitis A virus cellular receptor 1 (HAVCR1); adrenoceptor beta 3 (ADRB3); pannexin 3 (PANX3); G protein-coupled receptor 20 (GPR20); lymphocyte antigen 6 complex locus K9 (LY6K); olfactory receptor 51E2 (OR51E2); TCR gamma alternate reading frame protein (TARP); Wilms tumor protein (WT1); ETS translocation-variant gene 6 (ETV6-AML); sperm protein 17 (SPA17); X antigen family member 1A (XAGE1); angiopoietin-binding cell surface receptor 2 (Tie2); melanoma cancer testis antigen-1 (MAD-CT-1); melanoma cancer testis antigen-2 (MAD-CT-2); Fos-related antigen 1; p53 mutant; human telomerase reverse transcriptase (hTERT); sarcoma translocation breakpoints; melanoma inhibitor of apoptosis (ML-IAP); ERG (transmembrane protease serine 2 (TMPRSS2) ETS fusion gene); N-acetyl glucosaminyl-transferase V (NA17); paired box protein Pax-3 (PAX3); androgen receptor; cyclin B1; V-myc avian myelocytomatosis viral oncogene neuroblastoma derived homolog (MYCN); Ras homolog family member C (RhoC); cytochrome P450 1B1 (CYP1B1); CCCTC-binding factor (zinc finger protein)-like (BORIS); squamous cell carcinoma antigen recognized by T cells 3 (SART3); paired box protein Pax-5 (PAX5); proacrosin binding protein sp32 (OYTES1); lymphocyte-specific protein tyrosine kinase (LCK); A kinase anchor protein 4 (AKAP-4); synovial sarcoma, X breakpoint 2 (SSX2); CD79a; CD79b; CD72; leukocyte-associated immunoglobulin-like receptor 1 (LAIR1); Fc fragment of IgA receptor (FCAR); leukocyte immunoglobulin-like receptor subfamily member 2 (LILRA2); CD300 molecule-like family member f (CD300LF); C-type lectin domain family 12 member A (CLEC12A); bone marrow stromal cell antigen 2 (BST2); EGF-like module-containing mucin-like hormone receptor-like 2 (EMR2); lymphocyte antigen 75 (LY75); glypican-3 (GPC3); Fc receptor-like 5 (FCRL5); immunoglobulin lambda-like polypeptide 1 (IGLL1), and CD155;
the immune checkpoint proteins are selected from the group consisting of: 2B4, 4-1BB, 4-1BB ligand, B7-1, B7-2, B7H2, B7H3, B7H4, B7H6, BTLA, CD155, CD160, CD19, CD200, CD27, CD27 ligand, CD28, CD40, CD40 ligand, CD47, CD48, CTLA-4, DNAM-1, Galectin-9, GITR, GITR ligand, HVEM, ICOS, ICOS ligand, IDO1, KIR, 3DL3, LAG-3, OX40, OX40 ligand, PD-L1, PD-1, PD-L2, LAG3, PGK, SIRPα, TIM-3, TIGIT, and VSIG8.

In some embodiments, the one or more target molecules are one or more selected from: CD70, PSCA, and CEA.

In some embodiments, the engineered receptor is one or more selected from: CAR, TCR, and TAC.

13. The engineered cell according to item 12, wherein the engineered receptor that binds other molecules is a CEA-binding CAR. In some embodiments, the CEA-binding CAR, from N-terminus to C-terminus, comprises or consists of: a CEA-binding domain-a hinge region-a transmembrane region-a signal transduction domain, or a CEA-binding domain-a transmembrane region-a signal transduction domain. In some embodiments, the CEA is human CEA. In some embodiments, the CEA-binding domain is an antibody or an antigen-binding fragment of the antibody. In some embodiments, the antigen-binding fragment is scFv. In some embodiments, the signal transduction domain comprises or is a primary signal transduction domain. In some embodiments, the signal transduction domain comprises a primary signal transduction domain and a co-stimulatory domain. In some embodiments, the signal transduction domain consists of a primary signal transduction domain and a co-stimulatory domain. In some embodiments, the signal transduction domain comprises one or more co-stimulatory domains. In some embodiments, the CEA-binding CAR, from N-terminus to C-terminus, comprises or is: CEA scFv-8h-8TM-BBZ, wherein CEA scFv represents scFv consisting of a heavy chain variable region and a light chain variable region of an anti-CEA antibody, and "-" represents linkage via a peptide bond or a peptide chain. In some embodiments, the CEA scFv comprises or is an amino acid sequence as set forth in SEQ ID NO:5.

14. The engineered cell according to item 13, wherein the CEA-binding CAR comprises or is an amino acid sequence as set forth in SEQ ID NO:6, or a conservative substitution variant of SEQ ID NO:6, or an amino acid sequence having at least 85% (e.g., at least 85%, 86%, 87%, 88%, 89%, 90%, 91%, 92%, 93%, 94%, 95%, 96%, 97%, 98%, 99%, 99.1%, 99.2%, 99.3%, 99.4%, 99.5%, 99.6%, 99.7%, 99.8%, or 99.9%) sequence identity to SEQ ID NO:6.

15. The engineered cell according to item 12, wherein the engineered receptor that binds other molecules is a PSCA-binding CAR. In some embodiments, the PSCA-binding CAR, from N-terminus to C-terminus, comprises or consists of: a PSCA-binding domain-a hinge region-a transmembrane region-a signal transduction domain, or a PSCA-binding domain-a transmembrane region-a signal transduction domain. In some embodiments, the PSCA is human PSCA. In some embodiments, the PSCA-binding domain is an antibody or an antigen-binding fragment of the antibody. In some embodiments, the antigen-binding fragment is scFv. In some embodiments, the signal transduction domain comprises or is a primary signal transduction domain. In some embodiments, the signal transduction domain comprises a primary signal transduction domain and a co-stimulatory domain. In some embodiments, the signal transduction domain consists of a primary signal transduction domain and a co-stimulatory domain. In some embodiments, the signal transduction domain comprises one or more co-stimulatory domains. In some embodiments, the PSCA-binding CAR, from N-terminus to C-terminus, comprises or is: PSCA scFv-8h-8TM-BBZ, wherein PSCA scFv represents scFv consisting of a heavy chain variable region and a light chain variable region of an anti-PSCA antibody, and "-" represents linkage via a peptide bond or a peptide chain. In some embodiments, the PSCA scFv comprises or is an amino acid sequence as set forth in SEQ ID NO:8.

16. The engineered cell according to item 15, wherein the PSCA-binding CAR comprises or consists of an amino acid sequence as set forth in SEQ ID NO:9, or a conservative substitution variant of SEQ ID NO:9, or an amino acid sequence having at least 85% (e.g., at least 85%, 86%, 87%, 88%, 89%, 90%, 91%, 92%, 93%, 94%, 95%, 96%, 97%, 98%, 99%, 99.1%, 99.2%, 99.3%, 99.4%, 99.5%, 99.6%, 99.7%, 99.8%, or 99.9%) sequence identity to SEQ ID NO:9.

17. The engineered cell according to item 12, wherein the engineered receptor that binds other molecules is a CD70-binding CAR. In some embodiments, the CD70-binding CAR, from N-terminus to C-terminus, comprises or consists of: a CD70-binding domain-a hinge region-a transmembrane region-a signal transduction domain, or a CD70-binding domain-a transmembrane region-a signal transduction domain. In some embodiments, the CD70 is human CD70. In some embodiments, the CD70-binding domain is an antibody or an antigen-binding fragment of the antibody. In some embodiments, the antigen-binding fragment is scFv. In some embodiments, the signal transduction domain comprises or is a primary signal transduction domain. In some embodiments, the signal transduction domain comprises a primary signal transduction domain and a co-stimulatory domain. In some embodiments, the signal transduction domain consists of a primary signal transduction domain and a co-stimulatory domain. In some embodiments, the signal transduction domain comprises one or more co-stimulatory domains. In some embodiments, the CD70-binding CAR, from N-terminus to C-terminus, comprises or is: CD70 scFv-8h-8TM-BBZ, wherein CD70 scFv represents scFv consisting of a heavy chain variable region and a light chain variable region of an anti-CD70 antibody, and "-" represents linkage via a peptide bond or a peptide chain. In some embodiments, the CD70 scFv comprises or is an amino acid sequence as set forth in SEQ ID NO:35.

18. The engineered cell according to item 17, wherein the CD70-binding CAR comprises or is an amino acid sequence as set forth in SEQ ID N0:12, or a conservative substitution variant of SEQ ID N0:12, or an amino acid sequence having at least 85% (e.g., at least 85%, 86%, 87%, 88%, 89%, 90%, 91%, 92%, 93%, 94%, 95%, 96%, 97%, 98%, 99%, 99.1%, 99.2%, 99.3%, 99.4%, 99.5%, 99.6%, 99.7%, 99.8%, or 99.9%) sequence identity to SEQ ID NO:12.

The engineered cell according to any one of the preceding items, wherein the co-stimulatory domain comprises a signal transduction domain of one or more molecules selected from the group consisting of: CD27, CD28, 4-1BB, OX40, CD30, CD40, CD2, LFA-1, LIGHT, NKG2C, B7-H3, PD-1, ICOS, CDS, ICAM-1, GITR, BAFFR, LIGHTR, SLAMF7, CD7, NKp80 (KLRF1), CD160, CD19, CD4, CD8α, CD8β, IL2Rβ, IL2Ry, IL7Rα, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, TNFR2, TRANCE/RANKL, DNAM1, SLAMF4, CD84, CD96, CEACAM1, CRTAM, CD229, CD160, PSGL1, CD100, CD69, SLAMF6, SLAMF1, SLAMF8, CD162, LTBR, LAT, GADS, SLP-76, PAG/Cbp, NKp44, NKp30, NKp46, NKG2D, and a ligand that specifically binds to CD83.

The engineered cell according to any one of the preceding items, wherein the primary signal transduction domain comprises a signal transduction domain of one or more molecules selected from the following or consists of a signal transduction domain of one or more molecules selected from the following: CD3ζ, CD3γ, CD3δ, CD3ε, CD5, CD22, FcRγ, FcRβ, FcεRIγ, FcεRIβ, FcγRIIa, CD79α, CD79β, CD66d, DAP10, and DAP12.

The engineered cell according to any one of the preceding items, wherein the hinge region is a hinge region of IgG, IgD, CD8α or CD28, or a combination thereof.

The engineered cell according to any one of the preceding items, wherein the transmembrane domain comprises a transmembrane domain of one or more molecules selected from the following: TCRα, TCRβ, TCRγ, TCRδ, CD3ζ, CD3ε, CD3γ, CD3δ, CD4, CD5, CD6, CD7, CD8α, CD9, CD16, CD22, CD27, CD28, CD33, CD37, CD45, CD47, CD52, CD64, CD80, CD86, CD134, 4-1BB, CD152, CD154, CISH, and PD-1.

19. The engineered cell according to any one of items 11 to 18, which is derived from a T cell, NK cell, macrophage, DC cell, B cell, or a precursor cell thereof.

20. Use of the fusion protein according to any one of items 1 to 7, the engineered receptor according to item 8 or 9, the engineered nucleic acid molecule according to item 10, or the engineered cell according to any one of items 11 to 19 in the manufacture of a medicament for treating cancer. In some embodiments, the cancer is human cancer.

21. The use according to item 20, wherein the cancer is one or more selected from the following:
bladder cancer, blood cancer, bone cancer, bone marrow cancer, brain/nervous system cancer, breast cancer, colorectal cancer, esophageal cancer, gastrointestinal cancer, head cancer, kidney cancer, liver cancer, lung cancer, nasopharyngeal cancer, neck cancer, ovarian cancer, pancreatic cancer, prostate cancer, skin cancer, gastric cancer, testicular cancer, tongue cancer, and uterine cancer. In some embodiments, the cancer is human colorectal cancer. In some embodiments, the cancer is human bladder cancer. In some embodiments, the cancer is human renal clear cell adenocarcinoma.

In addition, the present application further provides a method for treating cancer, comprising administering to a subject in need thereof a therapeutically effective amount of the fusion protein according to any one of items 1 to 7, the engineered receptor according to item 8 or 9, the engineered nucleic acid molecule according to item 10, or the engineered cell according to any one of items 11 to 19. In some embodiments of the method for treating cancer, the cancer is one or more selected from the following:
bladder cancer, blood cancer, bone cancer, bone marrow cancer, brain/nervous system cancer, breast cancer, colorectal cancer, esophageal cancer, gastrointestinal cancer, head cancer, kidney cancer, liver cancer, lung cancer, nasopharyngeal cancer, neck cancer, ovarian cancer, pancreatic cancer, prostate cancer, skin cancer, gastric cancer, testicular cancer, tongue cancer, and uterine cancer. In some embodiments, the cancer is human colorectal cancer. In some embodiments, the cancer is human bladder cancer. In some embodiments, the cancer is human renal clear cell adenocarcinoma. In some embodiments, the subject or patient is a human patient.

In addition, the present application further provides a medicament for treating cancer, the medicament comprising the fusion protein according to any one of items 1 to 7, the engineered receptor according to item 8 or 9, the engineered nucleic acid molecule according to item 10, or the engineered cell according to any one of items 11 to 19. In some embodiments, the cancer is one or more selected from the following:

bladder cancer, blood cancer, bone cancer, bone marrow cancer, brain/nervous system cancer, breast cancer, colorectal cancer, esophageal cancer, gastrointestinal cancer, head cancer, kidney cancer, liver cancer, lung cancer, nasopharyngeal cancer, neck cancer, ovarian cancer, pancreatic cancer, prostate cancer, skin cancer, gastric cancer, testicular cancer, tongue cancer, and uterine cancer. In some embodiments, the cancer is human colorectal cancer. In some embodiments, the cancer is human bladder cancer. In some embodiments, the cancer is human renal clear cell adenocarcinoma. In some embodiments, the subject or patient is a human patient.

22. A method for prolonging an *in vivo* persistence period of CAR-T cells, comprising expressing the fusion protein according to any one of items 1 to 7 or the engineered receptor according to item 8 or 9 on a membrane of the CAR-T cells.

23. A method for enhancing *in vivo* expansion ability of CAR-T cells, comprising expressing the fusion protein according to any one of items 1 to 7 or the engineered receptor according to item 8 or 9 on a membrane of the CAR-T cells.

24. A method for enhancing *in vivo* killing ability of CAR-T cells against target cells, comprising expressing the fusion protein according to any one of items 1 to 7 or the engineered receptor according to item 8 or 9 on a membrane of the CAR-T cells.

In some embodiments, the *in vivo* refers to within a cancer patient or subject. In some embodiments, the *in vivo* refers to within a patient or subject with abnormal expression of CD155 in certain tissues and organs. In some embodiments, the cancer is one or more selected from the following:
bladder cancer, blood cancer, bone cancer, bone marrow cancer, brain/nervous system cancer, breast cancer, colorectal cancer, esophageal cancer, gastrointestinal cancer, head cancer, kidney cancer, liver cancer, lung cancer, nasopharyngeal cancer, neck cancer, ovarian cancer, pancreatic cancer, prostate cancer, skin cancer, gastric cancer, testicular cancer, tongue cancer, and uterine cancer.

In some embodiments, the CAR-T according to any one of items 22 to 24, from N-terminus to C-terminus, comprises a CAR having the following structure: an antigen binding domain-a hinge region-a transmembrane region-a signal transduction domain, or an antigen binding domain-a transmembrane region-a signal transduction domain. In some embodiments, the antigen binding domain comprises or is an antibody or an antigen-binding fragment thereof, such as scFv. In some embodiments, the antigen binding domain comprises or is a receptor-binding domain of a ligand, or a ligand-binding domain of a receptor. In some embodiments, the signal transduction domain comprises or is a primary signal transduction domain. In some embodiments, the signal transduction domain comprises a primary signal transduction domain and a co-stimulatory domain. In some embodiments, the signal transduction domain consists of a primary signal transduction domain and a co-stimulatory domain. In some embodiments, the signal transduction domain comprises one or more co-stimulatory domains. In some embodiments, the CAR-T according to any one of items 22 to 24, from N-terminus to C-terminus, comprises a CAR having the following structure: scFv-8h-8TM-BBZ, wherein scFv represents scFv consisting of a heavy chain variable region and a light chain variable region of an antibody, and "-" represents linkage via a peptide bond or a peptide chain.

2A self-cleaving peptide is a commonly used multi-gene expression strategy that enables co-expression of multiple genes at the translation level. This peptide was first discovered in foot-and-mouth disease virus (FMDV) in 1991, with an average length of 18-22 amino acids. To date, multiple distinct 2A peptides have been identified, derived from foot-and-mouth disease virus 2A (F2A), porcine teschovirus-1 2A (P2A), thosea asigna virus 2A (T2A), and equine rhinitis A virus 2A (E2A), respectively. Their main working principle is that during translation, when the ribosome recognizes the 2A peptide, it skips the synthesis of the glycyl-prolyl peptide bond therein and slips, thereby directly generating two independent proteins. The purpose of using the 2A peptide in the present application is to obtain the engineered immune cell expressing the CD226 fusion protein, ultimately validate the function of the engineered immune cell through experiments in examples, and further validate the unpredictable effect of the CD226 fusion protein on the engineered immune cell. In addition to the above 2A self-cleaving peptide, IRES (internal ribosome entry site, IRES) can also be used. IRES can recruit ribosomes to translate mRNA, allowing for independent expression of multiple proteins. The coding genes (also referred to as ORFs) for target molecules such as the CD226 fusion protein, at least one chimeric antigen receptor (CAR), and other fusion proteins are separated by the internal ribosome entry sites (IRES), and a single mRNA transcript will produce multiple proteins. The above-mentioned 2A peptide, IRES, and other small molecule sequences with similar functions can be collectively referred to as linkers. In addition to using the above linkers to achieve multi-gene expression, the above-mentioned engineered cell can also be obtained by introducing different genes separately into a target cell via transduction, or by introducing constructed vectors expressing different genes into a target gene via simultaneous transduction. Whether using linkers or separately constructing expression vectors of multiple genes for introduction into the target cells via transduction, the purpose of obtaining the engineered cell expressing CD226 can be finally achieved, and the function and unpredictable effects of the engineered cell are primarily based on the protein molecules it expresses and the final engineered cell itself.

The present application validates the function and efficacy of the engineered immune cell expressing the CD226 fusion protein and the engineered immune cell expressing the CD226 fusion protein and the chimeric antigen receptor. Although the examples herein are based on validation using the engineered T cell, the unpredictable effects of the engineered cell are primarily attributable to the CD226 fusion protein or the combination of the CD226 fusion protein and the CAR. Therefore, the unpredictable effects described in the present application likewise can be achieved in immune cells such as NK, DC, macrophages, NKT, and γδT.

### BRIEF DESCRIPTION OF THE DRAWINGS

FIG. 1 is a schematic diagram showing expression of an engineered cell expressing a CD226 fusion protein.
FIG. 2A and FIG. 2B show proliferation fold and viability of a CAR-T cell expressing the CD226 fusion protein, wherein FIG. 2A shows the proliferation fold, and FIG. 2B shows the viability.
FIG. 3 shows *in vitro* killing of CEA CAR-T expressing the CD226 fusion protein.
FIG. 4 shows cytokine secretion of the CEA CAR-T expressing the CD226 fusion protein *in vitro.*
FIG. 5 shows *in vivo* efficacy of the CEA CAR-T expressing the CD226 fusion protein in colorectal cancer tumor-bearing models.
FIG. 6 shows detection of CAR copy number in the colorectal cancer tumor-bearing mouse models treated with the CEA CAR-T expressing the CD226 fusion protein.
FIG. 7 shows proliferation fold of PSCA CAR-T cells expressing the CD226 fusion protein.
FIG. 8 shows *in vitro* killing of the PSCA CAR-T expressing the CD226 fusion protein.
FIG. 9 shows cytokine secretion of the PSCA CAR-T expressing the CD226 fusion protein *in vitro.*
FIG. 10 shows *in vivo* efficacy of the PSCA CAR-T expressing the CD226 fusion protein in bladder cancer tumor-bearing models.
FIG. 11 shows detection of CAR copy number in bladder cancer tumor-bearing mouse models treated with the PSCA CAR-T expressing the CD226 fusion protein.
FIG. 12 shows cytokine secretion of CD70 CAR-T expressing the CD226 fusion protein *in vitro.*

### DETAILED DESCRIPTION OF EMBODIMENTS

The present application relates to a transmembrane protein with an extracellular portion of CD226 as an antigen binding domain. The transmembrane protein, referred to as a fusion protein in the present application, comprises a CD226 extracellular region, a transmembrane domain, and a co-stimulatory domain. The fusion protein can be expressed on a surface of a CAR-T cell to enhance the tumor-killing ability of the CAR-T cell while prolonging the *in vivo* persistence of the CAR-T cell. The present application further provides an engineered cell expressing the fusion protein, such as the CAR-T cell, a nucleic acid molecule encoding the fusion protein, and use of the fusion protein, the nucleic acid molecule, and the engineered cell.

### Definitions

It should be understood that the present application is not limited to the aspects described herein, and certainly may vary. It should also be understood that, as used herein, the terms are used only to describe particular aspects and are not intended to be limiting, as the scope of the present application is limited only by the appended claims.

Unless otherwise defined, all technical and scientific terms used herein have the same meaning as commonly understood by those ordinarily skilled in the art to which the described technology belongs. All technical and patent publications cited herein are incorporated herein by reference in their entirety. Unless otherwise indicated, those skilled in the art would employ conventional techniques within the technical scope of the art, including tissue culture, immunology, molecular biology, microbiology, cell biology, and recombinant DNA.

The term "CD226" is an immune activator receptor, also known as DNAM-1, which competes with TIGIT (i.e., T cell immunoreceptor with Ig and ITIM domains, also known as WUCAM, Vstm3 or VSIG9) for the same set of ligands: CD155 (PVR or poliovirus receptor) and CD112 (nectin-2 or PVRL2). However, compared with the binding of CD226 to PVR, the binding affinity of CD225 to PVRL2 and PVRL3 is much weaker. Under natural conditions, CD226 is a glycoprotein predominantly expressed on the surface of NK cells, platelets, monocytes, and some T cells, and belongs to the Ig superfamily. CD226 consists of three domains: an extracellular domain, a transmembrane domain, and an intracellular domain. Herein, the extracellular domain comprises two immunoglobulin V-like domains and eight N-linked glycosylation sites. The intracellular domain comprises four tyrosine residues and one serine residue. When CD226 binds to its ligands, the CD226 molecule directionally moves to a lipid raft on the cell membrane and recruits intracellular signaling molecules (such as PTK and PKC), leading to phosphorylation of the four tyrosine residues and thus activating the cell. CD226 can mediate the adhesion of platelets and megakaryocytes to vascular endothelial cells and plays a role in megakaryocyte maturation. Exemplary CD226 includes human CD226. In some embodiments, CD226 is encoded by the gene with Gene ID 10666 in the NCBI database.

As used herein, the "CD226 extracellular region" may refer to any peptide fragment in the CD226 extracellular region, any truncation of the CD226 extracellular region, or the full-length CD226 extracellular region, so long as it retains the ability to bind to CD155. In some embodiments, the CD226 extracellular region comprises an amino acid sequence as set forth in SEQ ID NO:1 or 3. In some embodiments, the CD226 extracellular region comprises an amino acid sequence corresponding to SEQ ID NO:1 or 3 in any CD226 molecule. The term "corresponding to" refers to sequence alignment of positions of corresponding amino acids. After aligning the amino acid sequence of the any CD226 molecule with the amino acid sequence set forth in SEQ ID NO: 1 or 3 to have identical residues at as many positions as possible, by means such as introducing a gap into or deleting an amino acid from the amino acid sequence of the any CD226 molecule (exemplary methods include, e.g., BLAST, FASTA, and MEGLIGN), the amino acids of SEQ ID NO:1 or 3 are sequentially numbered starting from 1 from the first amino acid at amino (N)-terminus. In the amino acid sequence of the any CD226 molecule, the first amino acid and the last amino acid that are located at the same positions as the amino acid sequence set forth in SEQ ID NO:1 or 3 after alignment, as well as all amino acids between the first amino acid and the last amino acid, constitute the "amino acid sequence corresponding to SEQ ID NO: 1 or 3."

"CD155," also known as "PVR," i.e., poliovirus receptor, is also referred to as Necl5 and Tage4. CD155 is a cell surface adhesion molecule that is dramatically overexpressed in several human malignant tumors, while its expression is relatively low or absent in most healthy tissues. Its overexpression promotes invasion, migration, and proliferation of tumor cells, and is associated with poor prognosis and enhanced tumor progression.

"CD112," also known as "PVRL2," i.e., nectin-2, is a single-pass type I membrane protein with two Ig-like C2-type domains and one Ig-like V-type domain. It is a component of the plasma membrane of adherens junctions.

As used herein, the percentage of "identity," such as 85%, 90%, 91%, 92%, 93%, 94%, 95%, 96%, 97%, 98%, 98.5%, 99%, 99.5% identity, refers to the degree of similarity determined by sequence alignment between amino acid sequences or between nucleotide sequences, which is 85%, 90%, 91%, 92%, 93%, 94%, 95%, 96%, 97%, 98%, 98.5%, 99%, 99.5%. For example, after aligning two sequences to have identical residues at as many positions as possible by means such as introducing a gap, the percentage of identity is the determined proportion of the number of positions with identical bases or amino acid residues relative to the total number of positions. The percentage of "identity" can be determined using software programs known in the art. Preferably, alignment is performed using default parameters. One preferred alignment program is BLAST. Preferred programs include BLASTN and BLASTP. Details of these programs can be found at the following internet address: ncbi.nlm.nih.gov/cgi-bin/BLAST.

As used herein, a "variant" differs from a reference amino acid sequence by at least one amino acid, for example, by at least one amino acid addition, insertion, deletion, or substitution. For example, the amino acid substitution may be a conservative amino acid substitution, namely the substitution of an original corresponding amino acid with an amino acid having similar properties. "Conservative substitution" may refer to a polar-to-polar amino acid substitution, such as glycine (G, Gly), serine (S, Ser), threonine (T, Thr), tyrosine (Y, Tyr), cysteine (C, Cys), asparagine (N, Asn), and glutamine (Q, Gln); a nonpolar-to-nonpolar amino acid substitution, such as alanine (A, Ala), valine (V, Val), tryptophan (W, Trp), leucine (L, Leu), proline (P, Pro), methionine (M, Met), and phenylalanine (F, Phe); an acidic-to-acidic amino acid substitution, such as aspartic acid (D, Asp), glutamic acid (E, Glu); a basic-to-basic amino acid substitution, such as arginine (R, Arg), histidine (H, His), lysine (K, Lys); a charged amino acid-to-charged amino acid substitution, such as aspartic acid (D, Asp), glutamic acid (E, Glu), histidine (H, His), lysine (K, Lys), and arginine (R, Arg); a hydrophobic-to-hydrophobic amino acid substitution, such as alanine (A, Ala), leucine (L, Leu), isoleucine (I, Ile), valine (V, Val), proline (P, Pro), phenylalanine (F, Phe), tryptophan (W, Trp), and methionine (M, Met). In some other embodiments, the variant may also comprise non-conservative substitutions. In some embodiments, a "variant" of the amino acid sequence may have at least about 90%, 95%, 96%, 97%, 98%, or 99% sequence identity to the amino acid sequence. Compared with the amino acid sequence, a "variant" of the amino acid sequence may have an activity of at least 40%, 45%, 50%, 55%, 60%, 65%, 70%, 75%, 80%, 85%, 90%, 91%, 92%, 93%, 94%, 95%, 96%, 97%, 98%, 99%, or 100%, or a range constituted by any two of the preceding values. As used herein, a "conservative substitution variant" of a certain protein, polypeptide, or amino acid sequence refers to one in which one or more amino acid residues are subjected to amino acid substitution without altering the overall conformation and function of the protein or enzyme. This includes, but is not limited to, substitution of an amino acid in the amino acid sequence of a parent protein in the manner described in the preceding "conservative substitution". Accordingly, the similarity between two proteins or amino acid sequences with similar functions may vary. For example, a similarity (identity) ranges from 70% to 99% based on the MEGALIGN algorithm. "Conservative substitution variants" also encompasses polypeptides or enzymes determined by the BLAST or FASTA algorithm to have amino acid identity of 60% or more, preferably 75% or more, more preferably 85% or more, and most preferably 90% or more, and that have the same or substantially similar properties or functions as the natural or parent protein or enzyme.

As used herein, "amino acid" refers to any monomeric unit that can be incorporated into a peptide, polypeptide, or protein. As used herein, the term "amino acid" includes the following 20 natural or genetically encoded α-amino acids: alanine (Ala or A), arginine (Arg or R), asparagine (Asn or N), aspartic acid (Asp or D), cysteine (Cys or C), glutamine (Gln or Q), glutamic acid (Glu or E), glycine (Gly or G), histidine (His or H), isoleucine (Ile or I), leucine (Leu or L), lysine (Lys or K), methionine (Met or M), phenylalanine (Phe or F), proline (Pro or P), serine (Ser or S), threonine (Thr or T), tryptophan (Trp or W), tyrosine (Tyr or Y), and valine (Val or V). In some embodiments, when referring to any of the above natural amino acids, the present application also encompasses non-natural amino acids or amino acid analogs derived or modified therefrom. As used herein, "amino acid" also includes non-natural amino acids, modified amino acids (e.g., having a modified side chain and/or backbone), and amino acid analogs. For further illustration, an amino acid is generally an organic acid comprising a substituted or unsubstituted amino group, a substituted or unsubstituted carboxyl group, and one or more side chains or groups, or an analog of any of these groups. Exemplary side chains include, for example, mercapto, seleno, sulfonyl, alkyl, aryl, acyl, keto, azido, hydroxyl, hydrazino, cyano, halo, hydrazide, alkenyl, alkynyl, ether, borate, boronate, phospho, phosphonyl, phosphine, heterocycle, enone, imine, aldehyde, ester, thioacid, hydroxylamine, or any combination of these groups. Other representative amino acids include, but are not limited to, amino acids comprising photo-sensitive crosslinkers, metal-binding amino acids, spin-labeled amino acids, fluorescent amino acids, metal-containing amino acids, amino acids with novel functional groups, amino acids that interact covalently or non-covalently with other molecules, photocaged and/or photo-isomerizable amino acids, radioactive amino acids, amino acids comprising biotin or biotin analogs, glycosylated amino acids, other carbohydrate-modified amino acids, amino acids comprising polyethylene glycol or polyether, heavy atom-substituted amino acids, chemically cleavable and/or photo-cleavable amino acids, amino acids comprising carbon-linked sugars, redox-active amino acids, amino acids comprising aminothioic acids, and amino acids comprising one or more toxic moieties. Amino acids described in the present application include, but are not limited to: 20 natural amino acids and 2-aminoadipic acid (Aad), 3-aminoadipic acid (bAad), beta-alanine or beta-aminopropanoic acid (bAla), 2-aminobutyric acid (Abu), 4-aminobutyric acid or piperidic acid (4Abu), 6-aminocaproic acid (Acp), 2-aminoheptanoic acid (Ahe), 2-aminoisobutyric acid (Aib), 3-aminoisobutyric acid (bAib), 2-aminopimelic acid (Apm), 2,4-diaminobutyric acid (Dbu), methamphetamine (Des), 2,2'-diaminopimelic acid (Dpm), 2,3-diaminopropionic acid (Dpr), ethylglycine (EtGly), N-ethylasparagine (EtAsn), hydroxylysine (Hyl), allo-hydroxylysine (aHyl), 3-hydroxyproline (3Hyp), 4-hydroxyproline (4Hyp), isodesmosine (Ide), allo-isoleucine (aIle), N-methylglycine or sarcosine (MeGly), N-methylisoleucine (MeIle), 6-N-methyllysine (MeLys), N-methylvaline (MeVal), norvaline (Nva), norleucine (Nle), and ornithine (Orm). Thus, in some embodiments, after the mutation, the amino acid mutation at the position comprises a substitution mutation to any one of the above 20 natural amino acids and the above non-natural amino acids. In some embodiments, the amino acid mutation comprises a substitution mutation to any amino acid selected from the group consisting of: G, A, V, L, I, P, F, Y, W, S, T, C, M, N, Q, D, E, K, R, H, Aad, bAad, bAla, Abu, 4Abu, Acp, Ahe, Aib, bAib, Apm, Dbu, Des, Dpm, Dpr, EtGly, EtAsn, Hyl, aHyl, 3Hyp, 4Hyp, Ide, aIle, MeGly, MeIle, MeLys, MeVal, Nva, Nle, and Orm.

In the context of the present application, the terms "DNA" and "RNA" refer to single-stranded or double-stranded DNA or RNA molecules. Unless otherwise indicated, the terms "DNA" and "DNA molecule" refer to double-stranded DNA molecules composed of A, C, G, and/or T nucleotides, while the terms "RNA" and "RNA molecule" refer to single-stranded RNA molecules composed of A, C, G, and/or U nucleotides. As used herein, the A, C, G, T, and U nucleotides refer to nucleotides comprising adenine, guanine, cytosine, thymine, and uracil as their respective nitrogenous bases.

RNA molecules include coding RNA or non-coding RNA (ncRNA), such as pre-mRNA, mature mRNA, or long noncoding RNA (lncRNA).

As used herein, a "DNA-RNA hybrid molecule" refers to a molecule comprising a polynucleotide sequence composed of deoxyribonucleotides and ribonucleotides. The DNA-RNA hybrid molecule may be obtained by:
replacing one or more deoxyribonucleotides in DNA with ribonucleotides;
replacing one or more ribonucleotides in RNA with deoxyribonucleotides; or
de novo synthesis using deoxyribonucleotides and ribonucleotides as starting materials via biological or chemical synthesis. It should be noted that the manner of obtaining the DNA-RNA hybrid molecule is not limited to the above methods, and any DNA-RNA hybrid molecule obtained by any method falls within the scope of the "DNA-RNA hybrid molecule" as defined in the present application.

As used herein, if two nucleic acid molecules are described as having "identical genetic information," it means that the two nucleic acid molecules are complementary to each other, or comprise identical base sequences, or that after converting one or more thymines in the base sequence of one of the two nucleic acid molecules to uracils, a nucleic acid molecule having a base sequence identical to that of the other nucleic acid molecule can be obtained. Accordingly, any two of DNA, RNA, and DNA-RNA hybrid molecules may have identical genetic information. Herein, the term "base sequence" refers to the order of bases in a polynucleotide molecule. Those skilled in the art should understand that, unless otherwise indicated, a base sequence or polynucleotide sequence described in the present application, when used to describe a DNA sequence, may use "T" to represent thymine; however, when the base sequence or polynucleotide sequence is used to describe RNA (e.g., mRNA), "T" will be substituted by "U" (uracil). Thus, any DNA disclosed herein by a specific sequence identification number (SEQ ID NO) also discloses the RNA (e.g., mRNA or poly(A) tail) sequence that is complementary or corresponding to the DNA, wherein each "T" in the DNA sequence is replaced by "U."

As used herein, "encoding" means that i) a DNA sequence contains genetic information that can be transcribed into an RNA molecule, and/or ii) an RNA molecule contains genetic information that can be translated into an amino acid sequence. Thus, as used herein, a "coding sequence" may refer to a ribonucleotide (RNA) sequence in a pre-mRNA or mature mRNA that can be translated into a protein or a fragment thereof, or may refer to a complementary sequence of a deoxyribonucleic acid (DNA) sequence that serves as a template for transcribing the pre-mRNA or mature mRNA or a fragment thereof. In addition, the "coding sequence" of the present application may further comprise a polynucleotide sequence encoding a protein, a functional nucleic acid, or a fragment thereof, such as miRNA, shRNA, dsRNA, guide RNA, Poly(A) tail, 5'UTR, 3'UTR, etc. Herein, a DNA molecule containing genetic information that can be transcribed into an RNA molecule is referred to as a "coding nucleic acid" of the RNA molecule; an RNA molecule containing genetic information that can be translated into an amino acid sequence is referred to as a "coding nucleic acid" of the amino acid sequence.

Unless otherwise specified, "peptide," "polypeptide," and "protein" are used interchangeably in the present application and refer to any naturally active substance formed by covalent linkage of two or more amino acids via peptide bonds, which may or may not contain secondary or tertiary structures of a protein molecule.

As used herein, the term "contact" is used in its plain ordinary meaning and refers to a process of allowing at least two different substances to become sufficiently close to react, interact, or make physical contact. It is to be understood that the resulting reaction product may be generated directly from the reaction between the added reagents or from an intermediate of one or more added reagents, which intermediate may be produced in the reaction mixture. The term "contact" may include allowing two substances to react, interact or make physical contact, wherein the two substances may be, for example, an engineered receptor (or engineered nucleic acid molecule) and a cell as provided herein. In embodiments, contacting includes, for example, allowing the engineered nucleic acid molecule or engineered peptide in the present application to enter cells.

As used herein, *"in vivo* persistence period of CAR-T cells" refers to the duration during which CAR-T cells persist in a patient or subject, i.e., the period from administration of the CAR-T cells *in vivo* until their eventual disappearance from the body. It can be calculated or measured by comparing copy number of CARs. For example, after a specified period of *in vivo* use of the CAR-T, an increase in the copy number thereof can be used to indicate a prolonged *in vivo* persistence period of the CAR-T cells.

In addition, in the present application, 8h represents the CD8 hinge region, 8TM represents the CD8 transmembrane domain, BB represents the 4-1BB co-stimulatory domain (or known as the 4-1BB signal transduction domain), and Z or z represents the CD3ζ signal transduction domain.

Unless otherwise defined herein, all technical and scientific terms used herein have the same meanings as commonly understood by those ordinarily skilled in the art to which the application pertains.

### Fusion Protein

One aspect of the present application further provides a fusion protein comprising a CD226 extracellular region. The fusion protein is a transmembrane protein comprising an extracellular region, a transmembrane domain, and an intracellular region, and the fusion protein itself does not comprise a primary signal transduction domain. In some embodiments, the fusion protein, from N-terminus to C-terminus, comprises or consists of:
1) the CD226 extracellular region, the transmembrane domain, and a co-stimulatory domain; or
2) a signal peptide, the CD226 extracellular region, the transmembrane domain, and a co-stimulatory domain.

In some embodiments, the fusion protein, from N-terminus to C-terminus, comprises:
1) the CD226 extracellular region, the transmembrane domain, and a co-stimulatory domain; or
2) a secretory protein signal peptide, the CD226 extracellular region, the transmembrane domain, and a co-stimulatory domain.

Herein, the CD226 extracellular region and the transmembrane domain, as well as the transmembrane domain and the co-stimulatory domain are linked directly and/or indirectly. The term "direct" linkage or "directly linked" refers to linkage achieved only by chemical bonds, i.e., two proteins or polypeptides are not linked via other molecules. The chemical bonds may be non-covalent bonds (e.g., ionic bonds, hydrogen bonds, hydrophobic bonds or van der Waals bonds) or covalent bonds (e.g., peptide bonds). The term "indirect" linkage or "indirectly linked" refers to linkage via a linker, wherein two proteins or polypeptides linked to each other using a linker are respectively linked to one end of the linker via covalent or non-covalent bonds. The "linker" may be a peptide linker (i.e., a peptide chain, e.g., consisting of 1 to 50 amino acids or derivatives thereof) or a non-peptide linker. Moreover, the linker may be cleavable (i.e., capable of being hydrolyzed by enzymes in an organism, e.g., a mammalian organism) or non-cleavable. Exemplary non-peptide linkers include, but are not limited to, polyethylene glycol, polypropylene glycol, copolymers of ethylene glycol and propylene glycol, polyoxyethylene polyols, polyvinyl alcohol, polysaccharides, dextrans, polyvinyl ethers, biodegradable polymers, polymeric lipids, chitin and hyaluronic acid, or derivatives thereof, or combinations thereof.

In some embodiments, the CD226 extracellular region and the transmembrane domain comprise or are linked by a hinge region. The hinge region may be a hinge region of any molecule, or a portion between a recognition domain and a transmembrane domain of any membrane receptor molecule. As used herein, a "hinge region" is generally a flexible portion in a protein that can participate in the propagation of conformational changes, e.g., from an active site to other parts of the protein structure), including other subunits), such as the segment between immunoglobulin heavy chain CH1 and CH2. In some embodiments, the hinge region is a hinge region of one or more molecules selected from the following or consists of a hinge region of one or more molecules selected from the following: IgG, IgD, CD7, CD8α or CD28.

As used herein, the "co-stimulatory domain" is generally derived from a co-stimulatory receptor of an immune cell and provides a second signal or secondary intracellular signal for activating an immune cell (such as a T cell). In some embodiments, the co-stimulatory domain comprises one or more of the following or consists of one or more of the following: CD27, CD28, 4-1BB, OX40, CD30, CD40, CD2, LFA-1, LIGHT, NKG2C, B7-H3, PD-1, ICOS, CDS, ICAM-1, GITR, BAFFR, LIGHTR, SLAMF7, CD7, NKp80 (KLRF1), CD160, CD19, CD4, CD8α, CD8β, IL2Rβ, IL2Rγ, IL7Rα, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, TNFR2, TRANCE/RANKL, DNAM1, SLAMF4, CD84, CD96, CEACAM1, CRTAM, CD229, CD160, PSGL1, CD100, CD69, SLAMF6, SLAMF1, SLAMF8, CD162, LTBR, LAT, GADS, SLP-76, PAG/Cbp, NKp44, NKp30, NKp46, NKG2D, and a signal transduction domain of a ligand that specifically binds to CD83.

As used herein, the "transmembrane domain" is a part of a transmembrane protein that links intracellular and extracellular sides, and typically consists of α-helical fragments of amino acids, wherein these amino acids are hydrophobic in nature and thus are able to embed into the hydrophobic interior of the cell membrane. Generally, the "transmembrane domain" can participate in regulating and/or transducing transmembrane signals. An exemplary transmembrane domain may be a transmembrane domain of any one or more molecules selected from the following, or consist of a transmembrane domain of any one or more molecules selected from the following: ICOS, CD226, TCRα, TCRβ, TCRγ, TCRδ, CD3ζ, CD3ε, CD3γ, CD3δ, CD4, CD5, CD6, CD7, CD8α, CD9, CD16, CD22, CD27, CD28, CD33, CD37, CD45, CD47, CD52, CD64, CD80, CD86, CD134, 4-1BB, CD152, CD154, CISH, and PD-1.

In some embodiments, the extracellular region of the fusion protein, in addition to the CD226 extracellular region, further comprises one or more additional proteins, polypeptides or protein functional domains, and the CD226 extracellular region can form a protein complex by combining with the one or more additional proteins, polypeptides or protein functional domains in any manner. In some embodiments, the combining is such that the CD226 extracellular region is linked to the one or more additional proteins, polypeptides or protein functional domains directly and/or indirectly. In some embodiments, the C-terminus of the CD226 extracellular region in the fusion protein is linked to the N-terminus of the additional protein, polypeptide or protein functional domain. In some embodiments, the N-terminus of the CD226 extracellular region in the fusion protein is linked to the C-terminus of the additional protein, polypeptide or protein functional domain. In some embodiments, the N-terminus of the CD226 extracellular region in the fusion protein is linked to the N-terminus of the additional protein, polypeptide or protein functional domain. In some embodiments, the C-terminus of the CD226 extracellular region in the fusion protein is linked to the C-terminus of the additional protein, polypeptide or protein functional domain. In some embodiments, the CD226 extracellular region in the fusion protein and the multiple proteins, polypeptides or protein functional domains are linked in tandem with each other. In some embodiments, the C-terminus and/or N-terminus of the CD226 extracellular region in the fusion protein is linked to at least two or more additional proteins, polypeptides or protein functional domains, and the two or more additional proteins, polypeptides or protein functional domains are not linked in tandem. In some embodiments, the one or more proteins are a homologous protein of the CD226 extracellular region or a part of the homologous protein. In some embodiments, the one or more proteins are heterologous proteins of the CD226 extracellular region or parts of the heterologous protein.

In some embodiments, the one or more additional proteins, polypeptides or protein functional domains comprise a recognition polypeptide that specifically binds to one or more other proteins, and the one or more other proteins may be tumor antigen(s), for example, one or more selected from the group consisting of: prostate stem cell antigen (PSCA), carcinoembryonic antigen (CEA), CD123, thyroid stimulating hormone receptor (TSHR); CD171; CS-1; C-type lectin-like molecule-1; ganglioside GD3; Tn antigen; CD19; CD20; CD22; CD30; CD70; CD123; CD138; CD33; CD44; CD44v7/8; CD38; CD44v6; B7H3 (CD276), B7H6; KIT (CD117); interleukin 13 receptor subunit alpha (IL-13Rα); interleukin 11 receptor alpha (IL-11Rα); prostate specific membrane antigen (PSMA); NY-ESO-1; HIV-1 Gag; MART-1; gp100; tyrosinase; mesothelin; EpCAM; protease serine 21 (PRSS21); vascular endothelial growth factor receptor; Lewis(Y) antigen; CD24; platelet derived growth factor receptor beta (PDGFR-β); stage-specific embryonic antigen-4 (SSEA-4); cell surface associated mucin 1 (MUC1), MUC6; epidermal growth factor receptor family and mutants thereof (EGFR, EGFR2, ERBB3, ERBB4, EGFRvIII); neural cell adhesion molecule (NCAM); carbonic anhydrase IX (CAIX); LMP2; ephrin type-A receptor 2 (EphA2); fucosyl GM1; sialyl Lewis adhesion molecule (sLe); ganglioside GM3 (aNeu5Ac(2-3)bDGalp(1-4)bDGlcp(1-1)Cer); TGS5; high molecular weight melanoma-associated antigen (HMWMAA); O-acetyl-GD2 ganglioside (OAcGD2); folate receptor; tumor endothelial marker 1 (TEM1/CD248); tumor endothelial marker 7-related (TEM7R); Claudin 6, Claudin 18.2, Claudin 18.1; ASGPR1; CDH16; 5T4; 8H9; αvβ6 integrin; B cell maturation antigen (BCMA); CA9; kappa light chain; CSPG4; EGP2, EGP40; FAP; FAR; FBP; embryonic AchR; HLA-A1, HLA-A2; MAGEA1, MAGE3; KDR; MCSP; NKG2D ligand; PSC1; ROR1; Sp17; SURVIVIN; TAG72; TEM1; fibronectin; tenascin; carcinoembryonic variant of tumor necrosis region; G protein-coupled receptor class C group 5 member D (GPRC5D); chromosome X open reading frame 61 (CXORF61); CD97; CD179a; anaplastic lymphoma kinase (ALK); polysialic acid; placenta-specific 1 (PLAC1); hexasaccharide of globoH glycoceramide (GloboH); mammary gland differentiation antigen (NY-BR-1); uroplakin 2 (UPK2); hepatitis A virus cellular receptor 1 (HAVCR1); adrenoceptor beta 3 (ADRB3); pannexin 3 (PANX3); G protein-coupled receptor 20 (GPR20); lymphocyte antigen 6 complex locus K9 (LY6K); olfactory receptor 51E2 (OR51E2); TCR gamma alternate reading frame protein (TARP); Wilms tumor protein (WT1); ETS translocation-variant gene 6 (ETV6-AML); sperm protein 17 (SPA17); X antigen family member 1A (XAGE1); angiopoietin-binding cell surface receptor 2 (Tie2); melanoma cancer testis antigen-1 (MAD-CT-1); melanoma cancer testis antigen-2 (MAD-CT-2); Fos-related antigen 1; p53 mutant; human telomerase reverse transcriptase (hTERT); sarcoma translocation breakpoints; melanoma inhibitor of apoptosis (ML-IAP); ERG (transmembrane protease serine 2 (TMPRSS2) ETS fusion gene); N-acetyl glucosaminyl-transferase V (NA17); paired box protein Pax-3 (PAX3); androgen receptor; cyclin B1; V-myc avian myelocytomatosis viral oncogene neuroblastoma derived homolog (MYCN); Ras homolog family member C (RhoC); cytochrome P450 1B1 (CYP1B1); CCCTC-binding factor (zinc finger protein)-like (BORIS); squamous cell carcinoma antigen recognized by T cells 3 (SART3); paired box protein Pax-5 (PAX5); proacrosin binding protein sp32 (OYTES1); lymphocyte-specific protein tyrosine kinase (LCK); A kinase anchor protein 4 (AKAP-4); synovial sarcoma, X breakpoint 2 (SSX2); CD79a; CD79b; CD72; leukocyte-associated immunoglobulin-like receptor 1 (LAIR1); Fc fragment of IgA receptor (FCAR); leukocyte immunoglobulin-like receptor subfamily member 2 (LILRA2); CD300 molecule-like family member f (CD300LF); C-type lectin domain family 12 member A (CLEC12A); bone marrow stromal cell antigen 2 (BST2); EGF-like module-containing mucin-like hormone receptor-like 2 (EMR2); lymphocyte antigen 75 (LY75); glypican-3 (GPC3); Fc receptor-like 5 (FCRL5); immunoglobulin lambda-like polypeptide 1 (IGLL1), and CD155.

In some embodiments, the one or more additional proteins, polypeptides or protein functional domains comprise a recognition polypeptide that specifically binds to one or more other proteins, wherein the one or more other proteins may be immune checkpoint proteins, for example, one or more selected from the group consisting of:

2B4, 4-1BB, 4-1BB ligand, B7-1, B7-2, B7H2, B7H3, B7H4, B7H6, BTLA, CD155, CD160, CD19, CD200, CD27, CD27 ligand, CD28, CD40, CD40 ligand, CD47, CD48, CTLA-4, DNAM-1, Galectin-9, GITR, GITR ligand, HVEM, ICOS, ICOS ligand, IDO1, KIR, 3DL3, LAG-3, OX40, OX40 ligand, PD-L1, PD-1, PD-L2, LAG3, PGK, SIRPα, TIM-3, TIGIT, and VSIG8.

In some embodiments, the one or more additional proteins, polypeptides or protein functional domains comprise a recognition polypeptide that specifically binds to one or more other proteins, wherein the one or more other proteins include any one or more immune checkpoint proteins mentioned above, and any one or more tumor antigens mentioned above.

In some embodiments, the recognition polypeptide that specifically binds to one or more other proteins is an antibody or an antigen-binding fragment of the antibody, or a ligand or receptor that binds to the tumor antigen and/or immune checkpoint protein, or a fragment thereof.

### Engineered Receptor

The present application further provides an engineered receptor comprising the aforementioned fusion protein. In some embodiments, the engineered receptor further comprises primary signal transduction domain directly or indirectly linked to the C-terminus of the aforementioned fusion protein. In some embodiments, the engineered receptor further comprises a TCR binding domain. In some embodiments, the TCR binding domain is located between the extracellular region and the co-stimulatory domain of the aforementioned fusion protein.

In some embodiments, the engineered receptor is: a chimeric antigen receptor (CAR), a T cell receptor (TCR) or a T cell antigen coupler (TAC).

As used herein, the term "CAR," i.e., chimeric antigen receptor, comprises: i) an antigen binding domain that specifically recognizes one or more target antigens (e.g., tumor antigens) or target epitopes (e.g., tumor epitopes); ii) a transmembrane domain; and iii) an intracellular signal transduction domain. Herein, the "intracellular signal transduction domain" comprises a primary signal transduction domain and/or a co-stimulatory domain. In some embodiments, the intracellular signal transduction domain comprises a primary signal transduction domain derived from any molecule selected from the group consisting of: CD3ζ, CD3γ, CD3ε, CD3δ, FcRγ, FcRβ, CD5, CD22, CD79a, CD79b, CD66d, FcγRIIa, DAP10, and DAP12. In some embodiments, the primary signal transduction domain is derived from CD3ζ. As used herein, the "primary signal transduction domain" typically comprises immune-receptor tyrosine-based activation motifs (ITAMs), with a basic composition of YXXL/V, wherein Y is tyrosine, L/V refers to leucine or valine, and X can be any amino acid. Upon binding of the receptor to a corresponding ligand, tyrosine within the ITAM linked thereto can be phosphorylated under the action of a class of protein tyrosine kinases (PTKs) associated with the cell membrane, thereby recruiting other free intracellular protein kinases or adaptor proteins, and propagating activation signals into cells.

As used herein, the engineered "TCR," i.e., engineered T cell receptor, comprises: (a) an antigen binding domain (as used in the present application, the antigen binding domain comprises a domain that binds to an antigen, a ligand domain that binds to a receptor, or a receptor domain that binds to a ligand), which comprises an antigen binding fragment (e.g., sdAb, scFv, Fab, DARPin) that specifically recognizes one or more target antigens (e.g., tumor antigens) or target epitopes (e.g., tumor epitopes); (b) an optional first linker; (c) an optional extracellular antigen binding domain of a first TCR subunit (e.g., Cα, Cβ, Cδ, Cγ, CD3ε) or a part thereof; (d) a transmembrane domain of a second TCR subunit (e.g., TCRα, TCRβ); and (e) an intracellular signal transduction domain comprising a third TCR subunit (e.g., TCRα, TCRβ), wherein the first, second, and third TCR subunits are each independently any one selected from the group consisting of: TCRα, TCRβ, TCRγ, TCRδ, CD3ε, CD3γ, CD3δ, and CD3ζ. In some embodiments, the first, second, and third TCR subunits are the same (e.g., all CD3ε, all TCRα or all TCRβ). In some embodiments, the first, second, and third TCR subunits are different. In some embodiments, the engineered TCR further comprises a hinge domain located between C-terminus of the antigen binding domain and N-terminus of the transmembrane domain. In some embodiments, the hinge domain is derived from CD8α. In some embodiments, the antigen binding domain comprises the aforementioned engineered CD226 extracellular region. In some embodiments, the antigen binding domain is the aforementioned engineered CD226 extracellular region.

As used herein, "TAC," i.e., T cell antigen coupler, comprises (i) an antigen binding domain, (ii) a TCR binding domain (e.g., scFv), and (iii) a co-receptor domain (e.g., hinge, transmembrane and/or cytosolic region). See, e.g., Helsen et al. Nat Commun. 2018;9(1):3049. In some embodiments, the antigen binding domain comprises the aforementioned engineered CD226 extracellular region. In some embodiments, the antigen binding domain is the aforementioned engineered CD226 extracellular region. In some embodiments, the TAC comprises: (a) an antigen binding domain (as used in the present application, the antigen binding domain comprises a domain that binds to an antigen, a ligand domain that binds to a receptor, or a receptor domain that binds to a ligand), which comprises an antigen binding fragment (e.g., sdAb, scFv, Fab, DARPin) that specifically recognizes one or more target antigens (e.g., tumor antigens) or target epitopes (e.g., tumor epitopes); (b) an optional first linker; (c) an extracellular TCR binding domain (e.g., sdAb, scFv, Fab, DARPin) that specifically recognizes an extracellular antigen binding domain of a TCR subunit (e.g., CD3ε); (d) an optional second linker; (e) an optional extracellular antigen binding domain of a first TCR co-receptor (e.g., CD4, CD8) or a part thereof; (f) a transmembrane domain, comprising a transmembrane domain of a second TCR co-receptor (e.g., CD4, CD8); and (g) an optional intracellular signal transduction domain, comprising an intracellular signal transduction domain of a third TCR co-receptor (e.g., CD4, CD8), wherein the TCR subunit is any one or more selected from the group consisting of: CD3ε, CD3δ, CD3γ, TCRα, TCRβ, TCRγ, and TCRδ, and wherein the first, second, and third TCR co-receptors are each independently any one selected from the group consisting of: CD4, CD8, and CD28. In some embodiments, the first, second, and third TCR co-receptors are the same. In some embodiments, the first, second, and third TCR co-receptors are different. In some embodiments, the TAC further comprises a hinge domain (e.g., derived from CD8α) located between C-terminus of the antigen binding domain and N-terminus of the transmembrane domain. In some embodiments, the antigen binding domain comprises the aforementioned engineered CD226 extracellular region. In some embodiments, the antigen binding domain is the aforementioned engineered CD226 extracellular region.

As used herein, the term "antigen binding domain" encompasses the concepts of "ligand binding domain" and "receptor binding domain". It is typically located in the extracellular portion of a cell (especially immune cell) receptor and can specifically bind to a certain protein. The scope of the certain protein is not limited in any way. Therefore, in some embodiments, the certain protein is a certain receptor, and the "antigen binding domain" is a part of the ligand of the certain receptor that specifically recognizes the certain receptor; in some embodiments, the protein is a certain ligand, and the "antigen binding domain" is a part of the receptor of the certain ligand that specifically recognizes the ligand; in some embodiments, the protein is an antibody or an antigen binding domain of the antibody, such as a single-chain antibody (scFv), Fab, F(ab')2, Fab', Fv, Fd, dAb or diabody.

### Engineered Nucleic Acid Molecule

The present application further provides an engineered nucleic acid molecule encoding the aforementioned fusion protein or engineered receptor, the nucleic acid molecule comprising a coding sequence for a protein of interest. In some embodiments, after the engineered nucleic acid molecule is introduced into a suitable host cell, the engineered nucleic acid molecule can be transcribed and translated into the engineered receptor or fusion protein. The term "engineered nucleic acid molecule" is used to distinguish from a "natural nucleic acid molecule". A "natural nucleic acid molecule" refers to a nucleic acid molecule that exists in its natural form in nature. An "engineered nucleic acid molecule" imposes a limitation on the source or preparation method for the nucleic acid molecule, without imposing any limitation on its function or structure. Therefore, an engineered nucleic acid molecule can be used to refer to any nucleic acid molecule obtainable by any or multiple bioengineering means, which may have a polynucleotide sequence identical to that of a natural nucleic acid molecule, have identical modifications to that of a natural nucleic acid molecule, or even form a structure completely consistent with that of a natural nucleic acid molecule. However, the engineered nucleic acid molecule is distinguished from its corresponding natural nucleic acid molecule, or a natural nucleic acid molecule having the same polynucleotide sequence, at least in that the engineered nucleic acid molecule is not directly purified or extracted in its natural form from animals or plants naturally occurring in nature.

In some embodiments, the engineered nucleic acid molecule is an engineered DNA molecule. In some embodiments, the DNA molecule can be replicated and/or be expressed in cells. In some embodiments, the DNA molecule can be replicated and/or be expressed in eukaryotic cells. In some embodiments, the DNA molecule can be replicated and/or be expressed in prokaryotic cells. In some embodiments, the DNA molecule can be expressed in eukaryotic cells and can replicate in prokaryotic cells. Therefore, in addition to comprising the coding sequence for the protein of interest, the DNA molecule further comprises genetic operation or regulatory elements for replication and/or expression in prokaryotic and/or eukaryotic cells.

The necessary structural elements for enabling replication or efficient replication of the engineered DNA molecule in cells are known in the art, including, for example, an origin of replication (ORI). In some embodiments, the engineered DNA molecule further comprises a marker gene or a fragment thereof and/or a reporter gene or a fragment thereof, and unique restriction endonuclease sites allowing insertion of DNA elements, preferably restriction endonuclease sites in the form of a multiple cloning site (MCS). The marker gene facilitates the identification of cells containing a plasmid that comprises the marker gene, and may be selected from, for example, antibiotic resistance genes. Each restriction endonuclease site in the MCS can be specifically recognized by a different restriction endonuclease.

In some embodiments, the DNA molecule is a DNA plasmid. As used herein, the term "DNA plasmid" refers to a plasmid composed of double-stranded DNA molecules. In some embodiments, the "plasmid" is a circular DNA molecule. In some embodiments, the "plasmid" may also encompass linear DNA molecules. Specifically, the term "plasmid" further encompasses molecules obtained by linearizing a circular plasmid, for example, by cleaving the circular plasmid with a restriction endonuclease to convert the circular plasmid molecule into a linear molecule, as well as linear molecules capable of replicating in prokaryotes. A plasmid is capable of replication, i.e., amplification in a cell independent of genomic genetic information stored in the nucleoid or nucleoid-like structure of prokaryotic cells, and can be used for cloning, i.e., for amplifying genetic information in bacterial cells. Preferably, the DNA plasmid according to the application is a medium-copy or high-copy plasmid, more preferably a high-copy plasmid. Examples of such high-copy plasmids are vectors based on pUC, pTZ plasmids or any other plasmids that contain an ORI supporting high copy number of the plasmid (such as pMB1, pCoIE1) and the like.

In some embodiments, the engineered DNA molecule is a DNA molecule constituting a prokaryotic nucleoid or nucleoid-like structure or a fragment thereof, or a DNA molecule constituting the eukaryotic genome or a fragment thereof, i.e., the coding sequence for the protein of interest or its complementary sequence is capable of replicating along with the prokaryotic genome.

In some embodiments, the engineered DNA molecule can be transcribed into mRNA. In some embodiments, the engineered DNA molecule further comprises coding sequences for elements that can be used post-transcriptionally to initiate or regulate the expression of the protein, polypeptide or fragment thereof, the elements including but not limited to 5'UTR, 3'UTR, poly(A) tail (or polyadenylation signal) and the like. In some embodiments, the engineered DNA molecule comprises a coding sequence for at least one untranslated region (UTR). In some embodiments, the engineered DNA molecule comprises at least a coding sequence for 5'UTR and the coding sequence for the protein of interest. In some embodiments, the engineered DNA molecule, from 5' to 3', comprises sequentially at least a coding sequence for 5'UTR, the coding sequence for the protein of interest, a coding sequence for 3'UTR, a polyadenylation signal (or DNA sequence corresponding to the poly(A) tail sequence), and both ends of the coding sequence for the protein of interest may further comprise a start codon (at the 5' end) and a stop codon (at the 3' end), respectively, which are the first three nucleotides and the last three nucleotides of the mRNA molecule that are translatable. The 5'UTR generally comprises at least one ribosome binding site (RBS), such as the Shine-Dalgarno sequence in prokaryotes, or at least one translation initiation site, such as the Kozak sequence in eukaryotes. RBS promotes efficient and accurate translation of an mRNA molecule by recruiting ribosomes during initiation of translation. The activity of a given RBS can be optimized by varying its length and sequence or translation initiation site, as well as the distance to the start codon. Alternatively or optionally, the 5'UTR comprises internal ribosome entry sites or IRES. The 3'UTR may comprise one or more regulatory sequences, such as binding sites for amino acid sequences that enhance mRNA molecule stability, binding sites for regulatory RNA molecules (such as miRNA molecules), and/or signal sequences involved in the intracellular transport of mRNA molecules.

Based on the aforementioned embodiments, in some embodiments, the gene fragment of interest further comprises one or more additional regulatory sequences, such as a binding site for an amino acid sequence that enhances the mRNA molecule stability, a binding site for an amino acid sequence that enhances translation of the mRNA molecule, a regulatory element (such as riboswitch), and/or a nucleotide sequence that exerts a positive effect on translation initiation. Furthermore, within the 5'UTR, there are preferably no functional upstream open reading frames, out-of-frame upstream translation initiation sites, out-of-frame upstream start codons, and/or nucleotide sequences that generate a secondary structure that reduces or prevents translation. The presence of such nucleotide sequences in the 5'UTR may exert a negative effect on translation.

The coding sequence for the protein of interest comprises codons that can be translated into an amino acid sequence. Among all the codons comprised in the coding sequence for the protein of interest, all may be naturally occurring codons encoding amino acids, or some or all of them may be artificially synthesized codons. In some embodiments, some or all of the codons are codon-optimized. In some embodiments, some or all of the codons encode non-natural amino acids.

In some embodiments, the engineered DNA molecule further comprises, at the 5'-end side of the gene fragment of interest, structural elements necessary for initiating or regulating the transcription of the RNA, and such structural elements are known in the art. In some embodiments, the structural elements comprise at least a promoter. Promoters and sequences thereof are known in the art, including weak promoters, medium-strength promoters, strong promoters, mini promoters or core promoters and the like. In some specific embodiments, the promoter is a strong promoter. In some embodiments, the promoter can initiate transcription of the coding sequence for the protein of interest in prokaryotic cells. In some embodiments, the promoter can initiate transcription of the coding sequence for the protein of interest in eukaryotic cells. The "promoter" comprises at least one transcription recognition site and a downstream transcription factor binding site. The recognition and binding sites can interact with amino acid sequences that mediate or regulate transcription. The binding site is closer to the aforementioned gene fragment of interest than the recognition site. The binding site can be, for example, a Pribnow box in prokaryotes or a TATA box in eukaryotes. For example, in some embodiments, when a Pribnow box is used, the transcription recognition site can be located at about 35 bp upstream of the transcription start site, and the transcription factor binding site can be located at about 10 bp upstream of the transcription start site. In some embodiments, the promoter comprises at least one additional regulatory element, such as an AT-rich upstream element located about 40 and/or 60 nucleotides upstream of the transcription start site, and/or an additional regulatory element that enhances promoter activity located between the recognition site and the binding site. In some embodiments, the promoter is a strong promoter, i.e., the promoter comprises a sequence that promotes transcription of the aforementioned coding sequence for the protein of interest. Strong promoters are known to those skilled in the art, for example, OXB18, OXB19, and OXB20 promoters derived from the RecA promoter of *Escherichia coli,* or can be identified or synthesized by routine laboratory procedures. In some embodiments, the promoter is a T7 promoter. In some embodiments, additional regulatory elements, such as an enhancer comprised in a DNA plasmid that can promote transcription of the aforementioned coding sequence for the protein of interest, are further comprised upstream of the promoter.

In some embodiments, the eukaryotic cell is a yeast cell. In some embodiments, the DNA molecule is a yeast display vector.

In addition, the present application further provides an engineered RNA molecule encoding the aforementioned engineered receptor or fusion protein. In some embodiments, the engineered RNA molecule is obtained by transcription from the aforementioned engineered DNA molecule. In some embodiments, the engineered RNA molecule has the same sequence as an RNA molecule obtained by transcription from the aforementioned engineered DNA molecule. In some embodiments, the engineered RNA is mRNA. As used herein, "mRNA" (messenger RNA) refers to any naturally occurring, non-naturally occurring or modified RNA encoding at least one protein, polypeptide or fragment thereof. The mRNA is capable of being translated to produce the encoded protein, polypeptide, or fragment thereof *in vitro, in vivo, in situ* or *ex vivo.* Therefore, the mRNA can be mature mRNA or pre-mature mRNA, and the elements or structures that it must comprise or optionally comprise are known in the art. In some embodiments, the mRNA comprises coding sequences for multiple necessary functional components to express, regulate, or enhance the expression level of the protein, polypeptide, or fragment thereof. The functional components include, but are not limited to, 5' cap, 5'UTR, 3'UTR, and the like. Both 5'UTR and 3'UTR are typically transcribed from genomic DNA and are elements present in the pre-mature mRNA.

The term "5' cap" is located at the 5'-most end of mRNA and comprises a methylated guanylate that is linked to the 5' end of mRNA via pyrophosphate to form a 5',5'-triphosphate linkage with adjacent nucleotides. There are typically three types of 5' cap structures (m7G5'ppp5'Np, m7G5'ppp5'NmpNp, and m7G5'ppp5'NmpNmpNp), referred to as Type O, Type I, and Type II, respectively. Type O indicates that the ribose of the terminal nucleotide is unmethylated, Type I indicates that the ribose of one terminal nucleotide is methylated, and Type II indicates that the riboses of two terminal nucleotides are both methylated. In some embodiments, the 5' cap can be simultaneously added to the 5' end of a polynucleotide during an *in vitro* transcription reaction using the following chemical RNA cap analogs according to the manufacturer's protocol, producing a 5'-guanosine cap structure: 3'-O-Me-m7G(5')ppp(5')G [ARCA cap], G(5')ppp(5')A, G(5')ppp(5')G, m7G(5')ppp(5')A, m7G(5')ppp(5')G (New England BioLabs, Ipswich, MA), or m7G(5')ppp(5')(2'-OMeA)pG (CleanCap AG). For example, in some embodiments, vaccinia virus capping enzyme can be used to complete 5' capping of modified RNA after transcription to produce a Type O cap structure: m7G(5')ppp(5')G (New England BioLabs, Ipswich, MA). Both vaccinia virus capping enzyme and 2'-O-methyltransferase can be used to produce a Type I cap structure, yielding m7G(5')ppp(5')(2'-OMeA)pG. A Type II cap structure can be produced from the Type I cap structure by further 2'-O-methylation of the 5'-antepenultimate nucleotide using 2'-O-methyltransferase. A Type III cap structure can be produced from the Type II cap structure by further 2'-O-methylation of the 5'-preantepenultimate nucleotide using 2'-O-methyltransferase.

In some embodiments, the mRNA further comprises a stabilizing element. Stabilizing elements can include, for example, histone stem-loops. In some embodiments, the mRNA comprises a coding region, at least one histone stem-loop, and optionally a poly(A) sequence or a polyadenylation signal. The poly(A) sequence or polyadenylation signal should generally enhance the expression level of the encoded protein. In some embodiments, the mRNA comprises a combination of a poly(A) sequence or polyadenylation signal and at least one histone stem-loop, and although the two have an alternative mechanism in nature, their synergistic effect can increase protein expression to a level exceeding that is observed with either individual element. The synergistic effect of the combination of poly(A) and at least one histone stem-loop is independent of the order of elements or the length of the poly(A) sequence. In some embodiments, the histone stem-loop is typically derived from a histone gene and comprises two adjacent partially or fully reverse complementary sequences that form a loop through intramolecular base pairing, with a spacer region (composed of a short sequence) between them. Unpaired loop region is generally incapable of base pairing with either element of the stem-loop. The stability of a stem-loop structure generally depends on the length, the number of mismatches or bulges, and the base composition of the paired region. In some embodiments, wobble base pairing (non-Watson-Crick base pairing) may occur. In some embodiments, the at least one histone stem-loop sequence comprises 15 to 45 nucleotides in length.

In some embodiments, one or more AU-rich sequences of the mRNA can be removed. These sequences, sometimes referred to as AURES, are destabilizing sequences found in the 3'UTR. AURES can be removed from the mRNA. Alternatively, AURES can be retained in the mRNA.

In some embodiments, the mRNA is formulated in a lipid nanoparticle (LNP). In some embodiments, the lipid is mixed with the mRNA to form lipid nanoparticles. In some embodiments, the RNA is formulated in lipid nanoparticles. In some embodiments, the lipid nanoparticles are first formed as empty lipid nanoparticles and combined with or encapsulate the mRNA of a vaccine immediately prior to administration (e.g., within minutes to one hour).

The lipid nanoparticles generally comprise an ionizable lipid, a non-cationic lipid, sterol, and a PEG lipid component, as well as a target nucleic acid, such as the mRNA described above. The lipid nanoparticles of the present application can be produced using components, compositions, and methods as generally known in the art, with reference to, for example, PCT/US2016/052352, PCT/US2016/068300, PCT/US2017/037551, PCT/US2015/027400, PCT/US2016/047406, PCT/US2016000129, PCT/US2016/014280, PCT/US2016/014280, PCT/US2017/038426, PCT/US2014/027077, PCT/US2014/055394, PCT/US2016/52117, PCT/US2012/069610, PCT/US2017/027492, PCT/US2016/059575, and PCT/US2016/069491, all of which are incorporated herein by reference in their entirety.

In some embodiments, the engineered nucleic acid molecule further may be a DNA-RNA hybrid molecule, and the DNA-RNA hybrid molecule carries the same genetic information as the engineered DNA molecule or the engineered RNA molecule.

In some embodiments, the engineered nucleic acid molecule encodes an amino acid sequence as set forth in any one or more of SEQ ID NOs:1 to 12 or an amino acid sequence having at least 85% sequence identity to the amino acid sequence as set forth in any one or more of SEQ ID NOs:1 to 12. In some embodiments, the nucleic acid molecule comprises a polynucleotide sequence as set forth in any one or more of SEQ ID NOs:25 to 34 or a polynucleotide sequence having at least 85% sequence identity to the polynucleotide sequence as set forth in any one or more of SEQ ID NOs:25 to 34.

### Engineered Cell

The present application further provides an engineered cell, wherein the engineered cell expresses or comprises on its cell membrane the aforementioned fusion protein or the aforementioned engineered receptor, or wherein the engineered cell comprises the aforementioned engineered nucleic acid molecule.

In some embodiments, the engineered cell is an engineered immune cell. In some embodiments, the engineered cell is a T cell, NK cell, macrophage, DC cell, B cell, or a precursor cells thereof. In some embodiments, the engineered cell is a CAR-T or CAR-NK cell targeting one or more antigenic epitopes of CD155. In some embodiments, the engineered cell is a TCR-T cell targeting one or more antigenic epitopes of CD155. In some embodiments, the engineered cell is a TAC-T cell targeting one or more antigenic epitopes of CD155.

In some embodiments, the engineered cell is a CAR-T or CAR-NK cell targeting one or more different antigens, and it comprises the aforementioned fusion protein or engineered receptor, and one or more CARs targeting other tumor antigens. In some embodiments, the engineered cell is a CAR-T or CAR-NK cell targeting one or more different antigens, and it comprises the aforementioned fusion protein or engineered receptor, and one or more CARs targeting immune checkpoint proteins. In some embodiments, the engineered cell is a CAR-T or CAR-NK cell targeting one or more different antigens, and it comprises the aforementioned fusion protein or engineered receptor, one or more CARs targeting other tumor antigens, and one or more CARs targeting immune checkpoint proteins.

In some embodiments, the engineered cell is a TCR-T cell targeting one or more different antigens, and it comprises the aforementioned fusion protein or engineered receptor, wherein the TCR-T further comprises one or more TCRs targeting other tumor antigens. In some embodiments, the engineered cell is a TCR-T cell targeting one or more different antigens, and it comprises the aforementioned fusion protein or engineered receptor, wherein the TCR-T further comprises TCRs targeting one or more immune checkpoint proteins. In some embodiments, the engineered cell is a TCR-T cell targeting one or more different antigens, and it comprises the aforementioned fusion protein or engineered receptor, wherein the TCR-T further comprises one or more TCRs targeting other tumor antigens, and one or more TCRs targeting immune checkpoint proteins.

In some embodiments, the engineered cell is a TAC-T cell targeting one or more different antigens, and it comprises the aforementioned fusion protein or engineered receptor, wherein the TAC-T further comprises one or more TACs targeting other tumor antigens. In some embodiments, the engineered cell is a TAC-T cell targeting one or more different antigens, and it comprises the aforementioned fusion protein or engineered receptor, wherein the TAC-T further comprises TACs targeting one or more immune checkpoint proteins. In some embodiments, the engineered cell is a TAC-T cell targeting one or more different antigens, and it comprises the aforementioned fusion protein or engineered receptor, wherein the TAC-T further comprises one or more TACs targeting other tumor antigens, and one or more TACs targeting immune checkpoint proteins.

In some embodiments, the engineered cell expresses, or comprises on its cell membrane the aforementioned fusion protein or engineered receptor, and upon binding to CD155, the fusion protein or engineered receptor activates or inhibits a downstream signaling pathway of the fusion protein or engineered receptor.

In some embodiments, the engineered cell is selected from: T cell, NK cell, macrophage, DC cell, B cell, or a precursor cell thereof.

In some embodiments, the tumor antigen described herein is one or more selected from the following:
PSCA, CEA, CD123, TSHR, CD171, CS-1, C-type lectin-like molecule-1, ganglioside GD3, Tn antigen, CD19, CD20, CD22, CD30, CD70, CD123, CD138, CD33, CD44, CD44v7/8, CD38, CD44v6, B7H3 (CD276), B7H6, CD117, IL-13Rα, IL-11Rα, PSMA, NY-ESO-1, HIV-1 Gag, MART-1, gp100, tyrosinase, mesothelin, EpCAM, PRSS21, vascular endothelial growth factor receptor, Lewis(Y) antigen, CD24, PDGFR-β, SSEA-4, MUC1, MUC6, EGFR, EGFR2, ERBB3, ERBB4, EGFRvIII, NCAM, CAIX, LMP2, EphA2, fucosyl GM1, sLe, ganglioside GM3 (aNeu5Ac(2-3)bDGalp(1-4)bDGlcp(1-1)Cer), TGS5, HMWMAA, OAcGD2, folate receptor, CD248, TEM7R, Claudin 6, Claudin 18.2, Claudin 18.1, ASGPR1, CDH16, 5T4, 8H9, αvβ6 integrin, BCMA, CA9, kappa light chain, CSPG4, EGP2, EGP40, FAP, FAR, FBP, embryonic AchR, HLA-A1, HLA-A2, MAGEA1, MAGE3, KDR, MCSP, NKG2D ligand, PSC1, ROR1, Sp17, SURVIVIN, TAG72, TEM1, fibronectin, tenascin, carcinoembryonic variant of tumor necrosis region, GPRC5D, CXORF61, CD97, CD179a, ALK, polysialic acid, (PLAC1, GloboH, NY-BR-1, UPK2, HAVCR1, ADRB3, PANX3, GPR20, LY6K, OR51E2, TARP, WT1, ETV6-AML, SPA17, XAGE1, Tie2, MAD-CT-1, MAD-CT-2, Fos-related antigen 1, p53 mutant, hTERT, sarcoma translocation breakpoint, ML-IAP, ERG, NA17, PAX3, androgen receptor, cyclin B1, MYCN, RhoC, CYP1B1, BORIS, SART3, (PAX5, OYTES1, (LCK, AKAP-4, SSX2, CD79a, CD79b, CD72, LAIR1, FCAR, LILRA2, CD300LF, CLEC12A, BST2, EMR2, LY75, GPC3, FCRL5, IGLL1, and CD155.

In some embodiments, the immune checkpoint protein described herein is one or more selected from the following: 2B4, 4-1BB, 4-1BB ligand, B7-1, B7-2, B7H2, B7H3, B7H4, B7H6, BTLA, CD155, CD160, CD19, CD200, CD27, CD27 ligand, CD28, CD40, CD40 ligand, CD47, CD48, CTLA-4, DNAM-1, Galectin-9, GITR, GITR ligand, HVEM, ICOS, ICOS ligand, IDO1, KIR, 3DL3, LAG-3, OX40, OX40 ligand, PD-L1, PD-1, PD-L2, LAG3, PGK, SIRPα, TIM-3, CD226, and VSIG8.

In some embodiments, the engineered cell comprises the aforementioned fusion protein or engineered receptor, and a CAR, TCR or TAC that binds to CEA, PSCA or CD70. In some embodiments, the engineered cell comprises the aforementioned fusion protein or engineered receptor and a CEA-binding CAR. In some embodiments, the CEA-binding CAR has a structure as follows: CEA ScFv-8h-8TM-BBZ. In some embodiments, the engineered cell comprises the aforementioned fusion protein or engineered receptor and a PSCA-binding CAR. In some embodiments, the CEA-binding CAR has a structure as follows: PSCA ScFv-8h-8TM-BBZ. In some embodiments, the engineered cell comprises the aforementioned fusion protein or engineered receptor and a CD70-binding CAR. In some embodiments, the CD70-binding CAR has a structure as follows: CD70 ScFv-8h-8TM-BBZ. In the present application, CEA scFv, PSCA scFv, and CD70 scFv refer to scFvs formed by linking the heavy and light chains of antibodies targeting CEA, PSCA, and CD70, respectively.

### Use

The present application further provides use of the aforementioned engineered fusion protein, engineered receptor, engineered nucleic acid molecule, and engineered cell in the manufacture of a medicament for treating cancer. In some embodiments, the cancer is one or more selected from the group consisting of:

bladder cancer, blood cancer, bone cancer, bone marrow cancer, brain/nervous system cancer, breast cancer, colorectal cancer, esophageal cancer, gastrointestinal cancer, head cancer, kidney cancer, liver cancer, lung cancer, nasopharyngeal cancer, neck cancer, ovarian cancer, pancreatic cancer, prostate cancer, skin cancer, gastric cancer, testicular cancer, tongue cancer, and uterine cancer.

In some embodiments, tumor cells involved in the cancer highly express CD155.

In addition, the present application further provides use of the aforementioned fusion protein, engineered receptor, or engineered cell as a TIGIT antagonist, wherein the use prevents TIGIT on a surface of immune cells from binding to CD155, thereby reversing tumor immunosuppression caused by the binding of TIGIT to CD155. Therefore, in some embodiments, the fusion protein, engineered receptor, or engineered cell can be administered in combination with other anti-cancer agents.

### Examples

### Example 1: Functional Validation of CD226 Fusion Protein in CAR-T Cells Targeting CEA

### Cell Expansion and Viability Assay

Three vectors containing the gene sequences for CEA ScFv-8h-8TM-BBZ-P2A-CD226-28TM-28 (CEA CD226-CAR-T), CEA ScFv-8h-8TM-BBZ (CEA CAR-T), and CD226-28TM-28z (CD226-z) were constructed, and viruses were prepared. Herein, CEA scFv is scFv of an anti-CEA antibody, 8h represents a CD8 hinge region, 8TM represents a CD8 transmembrane domain, BB represents a 4-1BB co-stimulatory domain (or the 4-1BB signal transduction domain), Z or z represents a CD3ζ signal transduction domain, CD226 represents an extracellular region of CD226, 28TM represents a CD28 transmembrane domain, 28 represents a CD28 co-stimulatory domain (or the CD28 signal transduction domain), and P2A represents a 2A peptide. The specific sequences of the aforementioned structures used in the examples of the present application are as set forth in the Sequence Listing at the end of the text. Specifically, an amino acid sequence of CEA ScFv-8h-8TM-BBZ-P2A-CD226-28TM-28 is as set forth in SEQ ID NO:7, an amino acid sequence of CEA ScFv-8h-8TM-BBZ is as set forth in SEQ ID NO:6, and an amino acid sequence of the CD226 fusion protein (CD226-28TM-28) is as set forth in SEQ ID NO:2. Further, an amino acid sequence of CEA scFv is as set forth in SEQ ID NO:5, an amino acid sequence of 8h is as set forth in SEQ ID NO:13, an amino acid sequence of 8TM is as set forth in SEQ ID NO:14, an amino acid sequence of BB is as set forth in SEQ ID NO:15, an amino acid sequence of Z is as set forth in SEQ ID NO: 16, an amino acid sequence of P2A is as set forth in SEQ ID NO:24, an amino acid sequence of CD226 is as set forth in SEQ ID NO:1, an amino acid sequence of 28TM is as set forth in SEQ ID NO: 17, and an amino acid sequence of 28 is as set forth in SEQ ID NO: 18.

Herein, a nucleotide sequence of CEA CAR is as set forth in SEQ ID NO:29, a nucleotide sequence of CEA CD226 CAR is as set forth in SEQ ID NO:30, a nucleotide sequence of CD226 is as set forth in SEQ ID NO:25, and a nucleotide sequence of the CD226 fusion protein is as set forth in SEQ ID NO:26.

The specific virus preparation method is as follows:
In the present example, the lentivirus was packaged using the calcium phosphate method with reference to Molecular Cloning: A Laboratory Manual (Third Edition, by J. Sambrook et al.). Specifically, 293T cells were cultured in DMEM medium containing 10% FBS (w/v) to a good state. The aforementioned plasmids of interest were respectively mixed with three lentiviral packaging plasmids pMDLg/pRRE, pRSV-Rev, and pMD2.G at an appropriate ratio. CaCl₂ and 2×HBS were added, and after uniform mixing, the mixture was allowed to stand at room temperature and then added into the treated 293T cell culture medium. After 4-6 h, the medium was replaced with 10 mL of DMEM medium containing 10% FBS, and the cell supernatant was collected after 48 h or 72 h. The viral supernatant was collected and subjected to viral purification. The purified virus was aliquoted into 1.5 mL EP tubes and stored at -80 °C for later use.

The concentrated lentivirus was subjected to titer detection using 293T and/or CHO cells. 1×10⁵ 293T and/or CHO cells were inoculated into a 24-well plate. The concentrated virus was used to infect the 293T and/or CHO cells at volumes of 1, 2.5, and 10 µL/well (with the addition of DEAE transfection aid). Two days after infection, the infected 293T and/or CHO cells were collected for flow cytometry analysis. The total CAR expression was detected using Protein-L, and the viral titer was calculated by a formula as follows: Titer (TU/ml) = 1×10⁵× positive rate × dilution factor ÷ virus volume × 1000. The viral titers of the aforementioned CAR structures are shown in Table 1.

| **Name** | **Viral Titer** |
|---|---|
| CD226-z | 5.63E+08 |
| CEA CD226-CAR-T | 3.71E+08 |
| CEA CAR-T | 8.61E+08 |
| CD70 CD226-CAR-T | 9.23E+08 |
| CD70 CAR-T | 7.94E+08 |
| PSCA CD226-CAR-T | 5.46E+08 |
| PSCA CAR-T | 7.98E+08 |

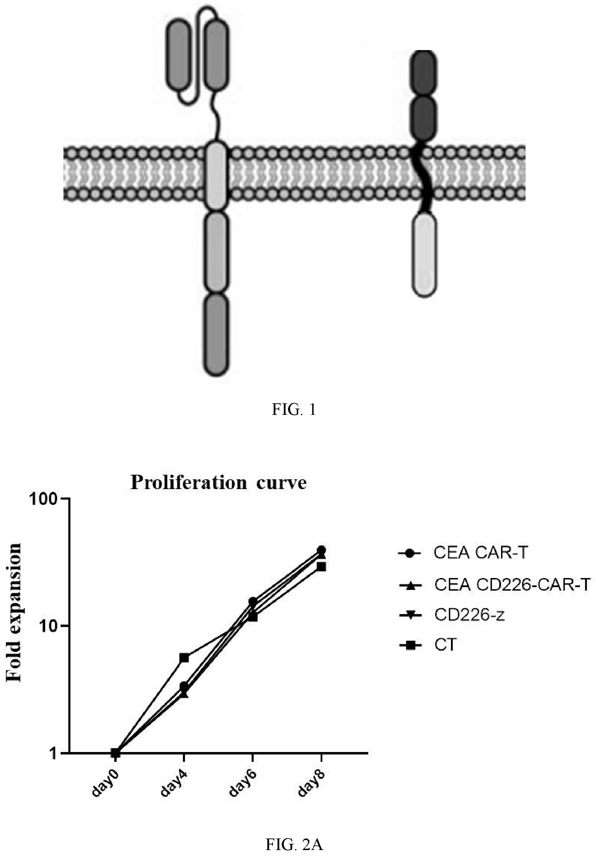

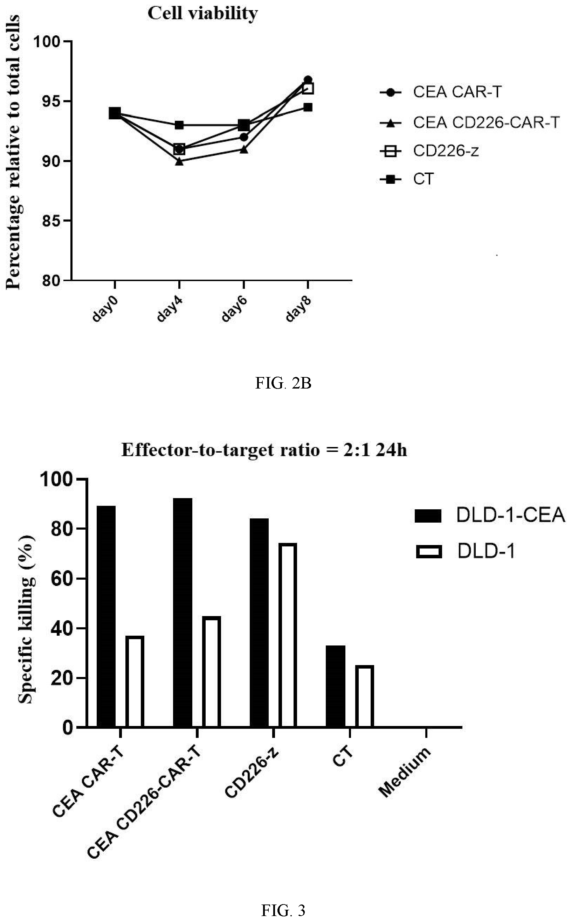

Lymphocytes were isolated using gradient centrifugation. After centrifugation, the second layer of white lymphocytes was collected and washed with normal saline to obtain human PBMCs. The obtained PBMCs were activated with anti-CD3 and anti-CD28 monoclonal antibodies for 24 h, and then the activated PBMCs were infected at a certain multiplicity of infection (MOI 3-5). On day 4 (d4), day 6 (d6), and day 8 (d8) after viral infection/transduction, the total cell count and viability were determined by cell counting. The results are shown in FIG. 2: FIG. 2A shows the cell expansion fold, and FIG. 2B shows the cell viability. These cells express the CD226 fusion protein. Herein, a schematic diagram of the CD226 fusion protein is shown on the right side of FIG. 1, comprising an extracellular domain, a transmembrane domain, and an intracellular domain. The extracellular domain is the CD226 polypeptide described in the present invention. The structure on the left side of FIG. 1 is a CAR structure targeting a tumor target such as CEA, mainly composed of an extracellular ScFv domain, a hinge region, a transmembrane domain, and an intracellular domain, and the intracellular domain may contain two domains.

The results in FIG. 2 show that the CEA CD226-CAR-T group exhibited no significant difference compared with the control CAR-T groups (CEA CAR-T and CD226-z groups) and the CT group, and all groups showed excellent expansion and viability.

### In vitro Efficacy Validation

DLD1-Luc-GFP is a human colorectal adenocarcinoma epithelial cell that expresses CD155 on the cell surface but is tested negative for membrane expression of CEA. On the basis of DLD1 cells, we overexpressed CEA molecules, making them double-positive cells expressing both CD155 and CEA. All the above cells were modified with Luc-GFP, hereinafter referred to as DLD1 and DLD1-CEA for short. DLD1-CEA (human colorectal adenocarcinoma epithelial cells, positive for CEA and CD155) and DLD1 (human colorectal adenocarcinoma epithelial cells, positive for CD155 and negative for CEA) were used as target cells, respectively. Effector cells were plated at an effector-to-target ratio of 2:1, and the killing ability of different groups against the target cells was detected after 24 h. The results are shown in FIG. 3. There was no significant difference in the killing of positive cells among the groups. However, CD226-z carrying the primary stimulation signal CD3ζ also killed CEA-negative DLD1, whereas CEA CD226-CAR-T with CD226 fusion protein and CEA CAR-T not expressing the CD226 fusion protein only killed CEA-positive DLD1-CEA cells, and showed no killing against CEA-negative DLD1 compared with the CT group. This indicates that the CD226 fusion protein described in the present application does not affect the specificity of the CAR when co-expressed with the CAR structure in immune cells, and the CD226 fusion protein does not directly cause killing of tumor cells by recognizing the CD155. The CD226-z structure added with the primary stimulation signal CD3ζ recognizes CD155 molecules expressed on the target cells and thus exerts killing against the target cells expressing the CD155.

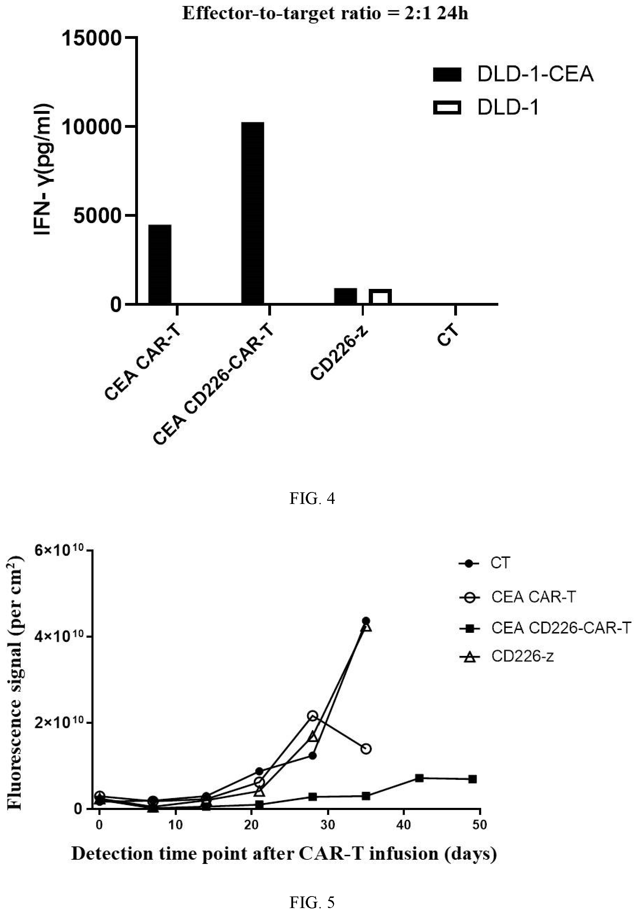

After 24 h, supernatant was collected to detect the IFN-γ secretion capacity of immune cells following stimulation by the target cells. The results are shown in FIG. 4. The IFN-γ secretion in the CEA CD226-CAR-T group was much higher than that in the CEA CAR-T group, the CT group, and the CD226-z group. This demonstrates that the combination of the CD226 fusion protein with the CAR can enhance the stimulation of the T cells by the CAR structure and improve the cytokine secretion capacity of the CAR-T cells. Such enhanced secretion capacity offers a significant advantage in addressing the insufficient efficacy of the CAR-T in current solid tumor therapy. In contrast, the CD226-z added with the primary stimulation signal CD3ζ did not exhibit this effect.

The above results indicate that the CEA CAR-T cells expressing the CD226 fusion protein (CEA CD226-CAR-T cells), compared with the control CEA CAR-T not expressing the CD226 fusion protein, maintained the specificity of the CEA CAR-T for the CEA target, and enhanced the activation capacity of the CAR-T, resulting in higher cytokine secretion.

### In vivo Antitumor Efficacy and Persistence Validation

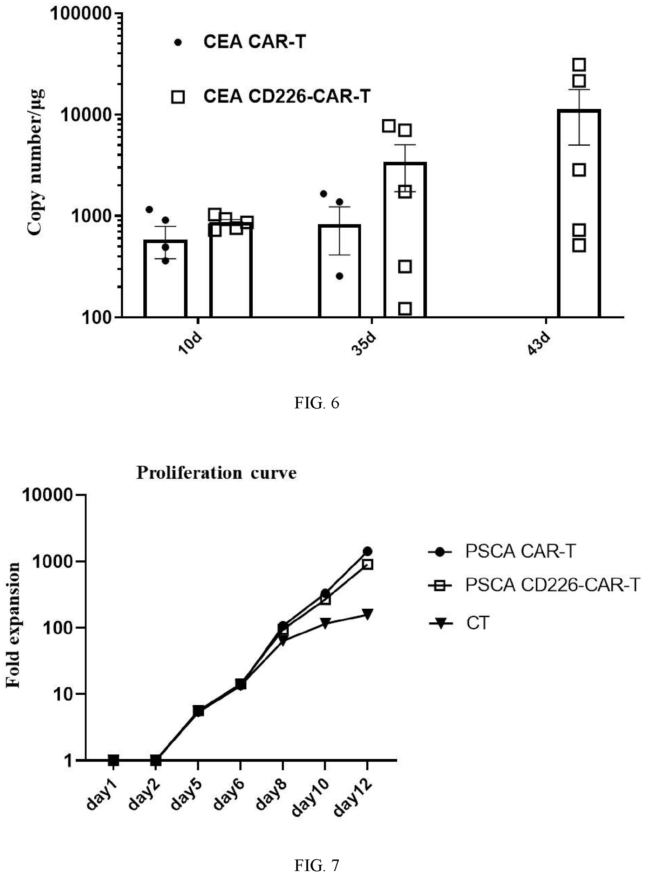

*In vivo* efficacy evaluation was conducted using 8-10 week old NCG immunodeficient mice. Tumors were established by intraperitoneal (i.p.) injection of 1e+6 DLD1-CEA-Luc-GFP cells per mouse. *In vivo* imaging was performed 7 days later, and mice were randomly grouped based on fluorescence values. Different groups were respectively administered i.p. with 2.0e+6 copies per mouse. *In vivo* imaging analysis and orbital blood collection were performed every 7 days for copy number detection. The results are shown in FIG. 5 and FIG. 6. FIG. 5 illustrates the effects of different CAR-T cells on tumors through fluorescence intensity in different tumors, with the CEA CD226-CAR-T exhibiting the optimal *in vivo* antitumor efficacy. FIG. 6 shows the CAR expression in the tumor-bearing mice at different time points after infusion of different CAR-T, as reflected by the copy number, further demonstrating the persistence of the CAR-T cells with different CAR structures in tissues of tumor-bearing organisms. Herein, the CEA CD226-CAR-T persisted in the blood of the tumor-bearing mice for at least 43 days, maintaining a relatively high copy number. The results indicate that the CEA CAR-T cells expressing the CD226 fusion protein (CEA CD226-CAR-T cells), compared with the control CEA CAR-T not expressing the CD226 fusion protein, prolonged mouse survival, inhibited tumor growth, delayed tumor recurrence, and exhibited superior *in vivo* expansion compared with the control CEA CAR-T group.

### Example 2: Functional Validation of CD226 Fusion Protein in CAR-T Cells Targeting PSCA

### Cell Preparation

The construction of CAR vectors and the preparation method for CAR-T were performed with reference to Example 1. Herein, the constructed CAR structures were as follows: three vectors with the gene sequences for PSCA ScFv-8h-8TM-BBZ-P2A-CD226-28TM-28 (PSCA CD226-CAR), PSCA ScFv-8h-8TM-BBZ (PSCA CAR-T), and CD226-28TM-28z (CD226-z), respectively. An amino acid sequence of PSCA ScFv-8h-8TM-BBZ-P2A-CD226-28TM-28 (PSCA CD226-CAR) is set forth in SEQ ID NO:10, an amino acid sequence of PSCA ScFv-8h-8TM-BBZ (PSCA CAR-T) is set forth in SEQ ID NO:9, and an amino acid sequence of the CD226 fusion protein (CD226-28TM-28) is set forth in SEQ ID NO:2. Herein, an amino acid sequence of PSCA scFv is set forth in SEQ ID NO:8, an amino acid sequence of 8h is set forth in SEQ ID NO:13, an amino acid sequence of 8TM is set forth in SEQ ID NO:14, an amino acid sequence of BB is set forth in SEQ ID NO:15, an amino acid sequence of Z is set forth in SEQ ID NO:16, an amino acid sequence of P2A is set forth in SEQ ID NO:24, an amino acid sequence of CD226 is set forth in SEQ ID NO:1, an amino acid sequence of 28TM is set forth in SEQ ID NO: 17, and an amino acid sequence of 28 is set forth in SEQ ID NO:18.

Herein, a nucleotide sequence of PSCA CAR is set forth in SEQ ID NO:31, a nucleotide sequence of PSCA CD226 CAR is set forth in SEQ ID NO:32, a nucleotide sequence of CD226 is set forth in SEQ ID NO:25, and a nucleotide sequence of the CD226 fusion protein is set forth in SEQ ID NO:26.

PBMC cells were activated with anti-CD3 and anti-CD28 monoclonal antibodies for 24 h, and then the activated PBMC cells were infected at a certain multiplicity of infection (MOI 3-5). On days 1, 2, 5, 6, 8, 10, and 12 after viral infection/transduction, the total cell count was determined by cell counting. The results are shown in FIG. 7: FIG. 7 shows the cell expansion fold.

The results show that the PSCA-targeting CAR-T group expressing the CD226 fusion protein exhibited no significant difference compared with the control CAR-T group and the CT group, and all groups showed excellent expansion and viability.

### In vitro Efficacy Validation

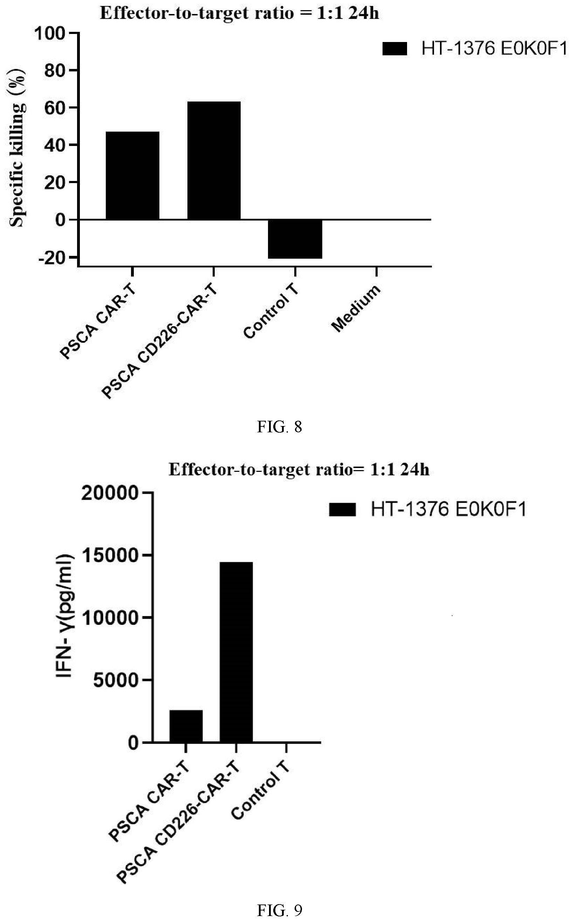

HT-1376 (human bladder cancer cell line, positive for PSCA and CD155) was used as target cells. Effector cells were plated at an effector-to-target ratio of 1:1, and the killing ability of different groups against the target cells was detected after 24 h. The results are shown in FIG. 8. The PSCA-targeting CAR-T group expressing the CD226 fusion protein (PSCA CD226-CAR-T), compared with the control CAR-T, exhibited no significant difference in killing of positive cells.

After 24 h, supernatant was collected to detect the IFN-γ secretion capacity of immune cells following stimulation by the target cells. The results are shown in FIG. 9. The IFN-γ secretion in the PSCA CD226-CAR-T group was much higher than that in the PSCA CAR-T group, approximately 5 times that in the PSCA CAR-T group.

The above results indicate that the PSCA CAR-T cells expressing the CD226 fusion protein (PSCA CD226-CAR-T cells), compared with the control PSCA CAR-T not expressing the CD226 fusion protein, exhibited enhanced CAR-T activation ability and showed higher cytokine secretion.

### In vivo Antitumor Efficacy and Persistence Validation

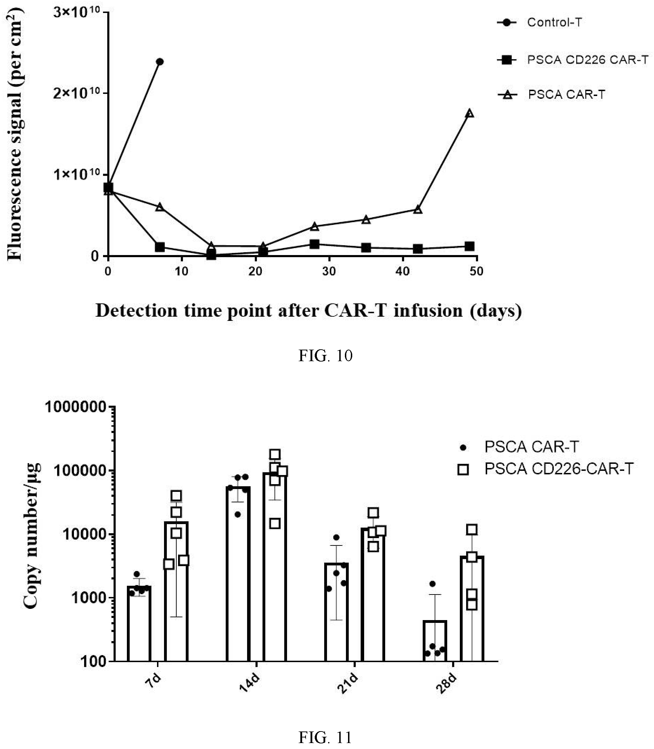

*In vivo* efficacy evaluation was conducted using 8-10 week old NCG immunodeficient mice. Tumors were established by i.p. injection of 3e+5 HT-1376-Luc-GFP cells per mouse. *In vivo* imaging was performed 14 days later, and the mice were randomly grouped according to fluorescence values. Different groups were respectively administered i.p. with 5.0e+5 CAR-T per mouse, and the total number of cells in the Control T group was consistent with that in the experimental groups. *In vivo* imaging analysis and orbital blood collection were performed every 7 days for copy number detection. The results are shown in FIG. 10 and FIG. 11. FIG. 10 shows the effects of different CAR-T cells on tumors via the fluorescence intensity of different tumors, wherein higher fluorescence intensity indicates larger tumor volume and higher tumor cell viability. FIG. 11 shows the CAR expression in the tumor-bearing mice at different time points after infusion of different CAR-T, as reflected by the copy number, further demonstrating the persistence of the CAR-T cells with different CAR structures in the tissues of tumor-bearing organisms. The results in FIG. 10 indicate that the PSCA CD226 CAR-T cells co-expressing the CD226 fusion protein had superior *in vivo* efficacy to the PSCA CAR-T not expressing the CD226 fusion protein; in the group receiving conventional PSCA CAR-T, tumor recurrence was observed on day 42 (the penultimate measurement point) after the CAR-T infusion, and the tumor cell fluorescence intensity significantly increased on day 49 (the last measurement point); in contrast, after infusion, the group treated with the PSCA CD226-CAR-T cells, targeting PSCA and expressing the CD226 fusion protein, maintained the antitumor efficacy up to day 49, demonstrating superior antitumor cells. The results in FIG. 11 indicate that on day 21, the CAR copy number in the group infused with the conventional PSCA CAR-T began to be significantly lower than that in the group infused with the PSCA CD226-CAR-T cells, targeting the PSCA and expressing the CD226 fusion protein, and by day 28, this difference had become markedly pronounced. Compared with the control PSCA CAR-T not expressing the CD226 fusion protein, the PSCA CAR-T cells expressing the CD226 fusion protein (PSCA CD226-CAR-T cells) inhibited tumor growth, delayed tumor recurrence, and showed superior *in vivo* expansion to the control PSCA CAR-T group.

Comparing the *in vitro* killing results of the CEA CAR-T and CEA CD226 CAR-T groups against CEA-negative, CD155-positive DLD1 cells in FIG. 3, it can be seen that the CD226 fusion protein did not directly kill the tumor cells. Comparing the groups in FIG. 3 and FIG. 5, it may also be found that the CAR structure CD226-z, designed with CD226 as the extracellular recognition domain, although exhibiting a killing function *in vitro,* did not show tumor-killing effect on the tumor-bearing mice; in contrast, the CEA CD226 CAR-T expressing the CD226 fusion protein was not only effective *in vivo* in mice, but also showed significantly higher efficacy than the CEA CAR-T alone. It can be seen that the improvement of CAR-T efficacy by the CD226 fusion protein does not solely depend on the extracellular region of CD226, nor does the CD226 fusion protein alone confer efficacy. Rather, the unpredictable result arises from the combined application of the fusion protein described in the present application with the CAR structure, resulting in a comprehensive effect that includes enhanced cytokine secretion upon CAR-T activation and increased *in vivo* persistence of CAR-T cells, etc.

The inventors also verified the combination of CAR structures targeting different targets, including CEA, PSCA, and CD70, with the fusion protein. Tumors associated with the above targets include, but are not limited to, various malignant tumors such as bladder cancer, blood cancer, bone cancer, bone marrow cancer, brain/nervous system cancer, breast cancer, colorectal cancer, esophageal cancer, gastrointestinal cancer, head cancer, kidney cancer, liver cancer, lung cancer, nasopharyngeal cancer, neck cancer, ovarian cancer, pancreatic cancer, prostate cancer, skin cancer, gastric cancer, testicular cancer, tongue cancer, and uterine cancer. Among tumor cells with the above multiple targets, the CAR-T cells comprising the CD226 fusion protein combined with the CAR showed superior efficacy.

### Example 3: Functional Validation of CD226 Fusion Protein in CAR-T Cells Targeting CD70

The construction of CAR vectors and the preparation method for CAR-T were performed with reference to Example 1. Herein, the constructed CAR structures were as follows: three vectors with gene sequences for CD70 ScFv-8h-8TM-BBZ-P2A-CD226-28TM-28 (CD70 CD226-CAR), CD70 ScFv-8h-8TM-BBZ (CD70 CAR-T), and CD226-28TM-28z (CD226-z), respectively. An amino acid sequence of CD70 ScFv-8h-8TM-BBZ-P2A-CD226-28TM-28 (CD70 CD226-CAR) is set forth in SEQ ID NO: 12, an amino acid sequence of CD70 ScFv-8h-8TM-BBZ (CD70 CAR-T) is set forth in SEQ ID NO:11, and an amino acid sequence of the CD226 fusion protein (CD226-28TM-28) is set forth in SEQ ID NO:2. Herein, an amino acid sequence of CD70 scFv is set forth in SEQ ID NO:35, an amino acid sequence of 8h is set forth in SEQ ID NO:13, an amino acid sequence of 8TM is set forth in SEQ ID NO:14, an amino acid sequence of BB is set forth in SEQ ID NO:15, an amino acid sequence of Z is set forth in SEQ ID NO:16, an amino acid sequence of P2A is set forth in SEQ ID NO:24, an amino acid sequence of CD226 is set forth in SEQ ID NO:1, an amino acid sequence of 28TM is set forth in SEQ ID NO:17, and an amino acid sequence of 28 is set forth in SEQ ID NO:18.

Herein, a nucleotide sequence of CD70 CAR is set forth in SEQ ID NO:33, a nucleotide sequence of CD70 CD226 CAR is set forth in SEQ ID NO:34, a nucleotide sequence of CD226 is set forth in SEQ ID NO:25, and a nucleotide sequence of the CD226 fusion protein is set forth in SEQ ID NO:26.

### In vitro Efficacy Validation

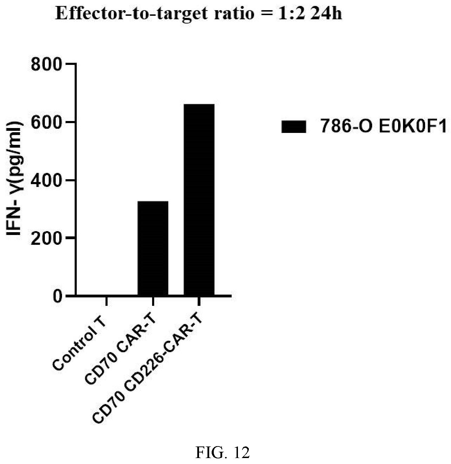

786-O (human renal clear cell adenocarcinoma cells, CD70-positive) was used as target cells. Effector cells were plated at an effector-to-target ratio of 1:2. After 24 h, supernatant was collected to assess the IFN-γ secretion capacity of immune cells following stimulation by the target cells. The results are shown in FIG. 12. The IFN-γ secretion in the CD70 CD226-CAR-T group was higher than that in the CD70 CAR-T group.

The above results indicate that the CD70 CAR-T cells expressing the CD226 fusion protein (CD70 CD226-CAR-T cells), compared with the control CD70 CAR-T not expressing the CD226 fusion protein, enhanced CAR-T activation capacity, and offered higher cytokine secretion.

The amino acid sequences and corresponding nucleic acid sequences of various proteins, polypeptides, and elements in the CAR structures used in the embodiments of the present application are set forth in the Sequence Listing below.

### Sequence Listing

| SEQ ID NO. | Name | |
|---|---|---|
| 1 | Amino acid | |
| | sequence of CD226 extracellular region | |
| 2 | CD226 fusion protein (CD226-28TM-28) | EEVLWHTSVPFAENMSLECVYPSMGILTQVEWFKIGTQQDSIAIFSPTH GMVIRKPYAERVYFLNSTMASNNMTLFFRNASEDDVGYYSCSLYTYP QGTWQKVIQVVQSDSFEAAVPSNSHIVSEPGKNVTLTCQPQMTWPVQ AVRWEKIQPRQIDLLTYCNLVHGRNFTSKFPRQIVSNCSHGRWSVIVIP DVTVSDSGLYRCYLQASAGENETFVMRLTVAEGKTDNQYTLFVATRF WVLVVVGGVLACYSLLVTVAFIIFWVRSKRSRGGHSDYMNMTPRRPG PTRKHYQPYAPPRDFAAYRS |
| 3 | Amino acid sequence of CD226 extracellular region with signal peptide | MDYPTLLLALLHVYRALCEEVLWHTSVPFAENMSLECVYPSMGILTQ VEWFKIGTQQDSIAIFSPTHGMVIRKPYAERVYFLNSTMASNNMTLFFR NASEDDVGYYSCSLYTYPQGTWQKVIQVVQSDSFEAAVPSNSHIVSEP GKNVTLTCQPQMTWPVQAVRWEKIQPRQIDLLTYCNLVHGRNFTSKF PRQIVSNCSHGRWSVIVIPDVTVSDSGLYRCYLQASAGENETFVMRLT VAEGKTDNQYTLFVA |
| 4 | CD226 signal peptide | MDYPTLLLALLHVYRALC |
| 5 | CEA scFv | DIQLTQSPSSLSASVGDRVTITCKASQDVGTSVAWYQQKPGKAPKLLIY WTSTRHTGVPSRFSGSGSGTDFTFTISSLQPEDIATYYCQQYSLYRSFGQ GTKVEIKGSTSGSGKPGSGEGSTKGEVQLVESGGGVVQPGRSLRLSCS ASGFDFTTYWMSWVRQAPGKGLEWIGEIHPDSSTINYAPSLKDRFTISR DNAKNTLFLQMDSLRPEDTGVYFCASLYFGFPWFAYWGQGTPVTVSS |
| 6 | CEA CAR (CEA ScFv-8h-8TM-BBZ) | DIQLTQSPSSLSASVGDRVTITCKASQDVGTSVAWYQQKPGKAPKLLIY WTSTRHTGVPSRFSGSGSGTDFTFTISSLQPEDIATYYCQQYSLYRSFGQ GTKVEIKGSTSGSGKPGSGEGSTKGEVQLVESGGGVVQPGRSLRLSCS ASGFDFTTYWMSWVRQAPGKGLEWIGEIHPDSSTINYAPSLKDRFTISR DNAKNTLFLQMDSLRPEDTGVYFCASLYFGFPWFAYWGQGTPVTVSS LETTTPAPRPPTPAPTIASQPLSLRPEACRPAAGGAVHTRGLDFACDIYI WAPLAGTCGVLLLSLVITLYCKRGRKKLLYIFKQPFMRPVQTTQEEDG CSCRFPEEEEGGCELRVKFSRSADAPAYKQGQNQLYNELNLGRREEYD VLDKRRGRDPEMGGKPRRKNPQEGLYNELQKDKMAEAYSEIGMKGE RRRGKGHDGLYQGLSTATKDTYDALHMQALPPR |
| 7 | CEA CD226CAR (CEA ScFv - 8h-8TM- | DIQLTQSPSSLSASVGDRVTITCKASQDVGTSVAWYQQKPGKAPKLLIY WTSTRHTGVPSRFSGSGSGTDFTFTISSLQPEDIATYYCQQYSLYRSFGQ GTKVEIKGSTSGSGKPGSGEGSTKGEVQLVESGGGVVQPGRSLRLSCS ASGFDFTTYWMSWVRQAPGKGLEWIGEIHPDSSTINYAPSLKDRFTISR |
| | BBZ-P2A-CD226-28TM-28) | DNAKNTLFLQMDSLRPEDTGVYFCASLYFGFPWFAYWGQGTPVTVSS LETTTPAPRPPTPAPTIASQPLSLRPEACRPAAGGAVHTRGLDFACDIYI WAPLAGTCGVLLLSLVITLYCKRGRKKLLYIFKQPFMRPVQTTQEEDG CSCRFPEEEEGGCELRVKFSRSADAPAYKQGQNQLYNELNLGRREEYD VLDKRRGRDPEMGGKPRRKNPQEGLYNELQKDKMAEAYSEIGMKGE RRRGKGHDGLYQGLSTATKDTYDALHMQALPPRVDGSGATNFSLLKQ AGDVEENPGPMDYPTLLLALLHVYRALCEEVLWHTSVPFAENMSLEC VYPSMGILTQVEWFKIGTQQDSIAIFSPTHGMVIRKPYAERVYFLNSTM ASNNMTLFFRNASEDDVGYYSCSLYTYPQGTWQKVIQVVQSDSFEAA VPSNSHIVSEPGKNVTLTCQPQMTWPVQAVRWEKIQPRQIDLLTYCNL VHGRNFTSKFPRQIVSNCSHGRWSVIVIPDVTVSDSGLYRCYLQASAGE NETFVMRLTVAEGKTDNQYTLFVATRFWVLVVVGGVLACYSLLVTV AFIIFWVRSKRSRGGHSDYMNMTPRRPGPTRKHYQPYAPPRDFAAYRS |
| 8 | PSCA scFv | DIEMTQSPSSLSASVGDRVTITCRASQSISSHLNWYQQKSGKAPKLLIY AASSLQGGVPSRFSGSGSGTDFTLTISSLQPEDFATYYCQQSYSPPWTFG QGTKLELKRGSTSGSGKPGSGEGSTKGEVQLVQSGAEVKKPGSSVKVS CKASGGTFSSYAISWVRQAPGQGLEWMGGIIPIFGTANYAQKFQGRVTI TADESTSTAYMELSSLRSEDTAVYYCARDTMIVANAFDIWGQGTTVTV SS |
| 9 | PSCA CAR (PSCA ScFv-8h-8TM-BBZ) | DIEMTQSPSSLSASVGDRVTITCRASQSISSHLNWYQQKSGKAPKLLIY AASSLQGGVPSRFSGSGSGTDFTLTISSLQPEDFATYYCQQSYSPPWTFG QGTKLELKRGSTSGSGKPGSGEGSTKGEVQLVQSGAEVKKPGSSVKVS CKASGGTFSSYAISWVRQAPGQGLEWMGGIIPIFGTANYAQKFQGRVTI TADESTSTAYMELSSLRSEDTAVYYCARDTMIVANAFDIWGQGTTVTV SSLETTTPAPRPPTPAPTIASQPLSLRPEACRPAAGGAVHTRGLDFACDI YIWAPLAGTCGVLLLSLVITLYCKRGRKKLLYIFKQPFMRPVQTTQEED GCSCRFPEEEEGGCELRVKFSRSADAPAYKQGQNQLYNELNLGRREEY DVLDKRRGRDPEMGGKPRRKNPQEGLYNELQKDKMAEAYSEIGMKG ERRRGKGHDGLYQGLSTATKDTYDALHMQALPPR |
| 10 | PSCA CD226 CAR (PSCA ScFv-8h-8TM-BBZ-P2A-CD226-28TM-28) | DIEMTQSPSSLSASVGDRVTITCRASQSISSHLNWYQQKSGKAPKLLIY AASSLQGGVPSRFSGSGSGTDFTLTISSLQPEDFATYYCQQSYSPPWTFG QGTKLELKRGSTSGSGKPGSGEGSTKGEVQLVQSGAEVKKPGSSVKVS CKASGGTFSSYAISWVRQAPGQGLEWMGGIIPIFGTANYAQKFQGRVTI TADESTSTAYMELSSLRSEDTAVYYCARDTMIVANAFDIWGQGTTVTV SSLETTTPAPRPPTPAPTIASQPLSLRPEACRPAAGGAVHTRGLDFACDI YIWAPLAGTCGVLLLSLVITLYCKRGRKKLLYIFKQPFMRPVQTTQEED GCSCRFPEEEEGGCELRVKFSRSADAPAYKQGQNQLYNELNLGRREEY DVLDKRRGRDPEMGGKPRRKNPQEGLYNELQKDKMAEAYSEIGMKG |
| | | ERRRGKGHDGLYQGLSTATKDTYDALHMQALPPRVDGSGATNFSLLK QAGDVEENPGPMDYPTLLLALLHVYRALCEEVLWHTSVPFAENMSLE CVYPSMGILTQVEWFKIGTQQDSIAIFSPTHGMVIRKPYAERVYFLNST MASNNMTLFFRNASEDDVGYYSCSLYTYPQGTWQKVIQVVQSDSFEA AVPSNSHIVSEPGKNVTLTCQPQMTWPVQAVRWEKIQPRQIDLLTYCN LVHGRNFTSKFPRQIVSNCSHGRWSVIVIPDVTVSDSGLYRCYLQASAG ENETFVMRLTVAEGKTDNQYTLFVATRFWVLVVVGGVLACYSLLVTV AFIIFWVRSKRSRGGHSDYMNMTPRRPGPTRKHYQPYAPPRDFAAYRS |
| 11 | CD70 CAR (CD70 ScFv-8h-8TM-BBZ) | DIQMTQSPSSLSASVGDRVTITCRASQSISSYLNWYQQKPGKAPKLLIFA ASSLQSGVPSRFSGSGSGTDFTLTISSLQPEDFATYYCQQSYSTPWTFGQ GTKLDIKRGSTSGSGKPGSGEGSTKGEVQLVQSGPEVKKPGASVKVSC KASGYTFTDYYMNWVRQMHGKGLEWMGVINPYNGGTDYNQKFKGR VTITADKSTSTAYMELSSLRSEDTAVYYCARSVYDYPFDYWGQGTLVT VSSLETTTPAPRPPTPAPTIASQPLSLRPEACRPAAGGAVHTRGLDFACD IYIWAPLAGTCGVLLLSLVITLYCKRGRKKLLYIFKQPFMRPVQTTQEE DGCSCRFPEEEEGGCELRVKFSRSADAPAYKQGQNQLYNELNLGRREE YDVLDKRRGRDPEMGGKPRRKNPQEGLYNELQKDKMAEAYSEIGMK GERRRGKGHDGLYQGLSTATKDTYDALHMQALPPR |
| 12 | CD70 CD226CAR (CD70 ScFv-8h-8TM-BBZ-P2A-CD226-28TM-28) | DIQMTQSPSSLSASVGDRVTITCRASQSISSYLNWYQQKPGKAPKLLIFA ASSLQSGVPSRFSGSGSGTDFTLTISSLQPEDFATYYCQQSYSTPWTFGQ GTKLDIKRGSTSGSGKPGSGEGSTKGEVQLVQSGPEVKKPGASVKVSC KASGYTFTDYYMNWVRQMHGKGLEWMGVINPYNGGTDYNQKFKGR VTITADKSTSTAYMELSSLRSEDTAVYYCARSVYDYPFDYWGQGTLVT VSSLETTTPAPRPPTPAPTIASQPLSLRPEACRPAAGGAVHTRGLDFACD IYIWAPLAGTCGVLLLSLVITLYCKRGRKKLLYIFKQPFMRPVQTTQEE DGCSCRFPEEEEGGCELRVKFSRSADAPAYKQGQNQLYNELNLGRREE YDVLDKRRGRDPEMGGKPRRKNPQEGLYNELQKDKMAEAYSEIGMK GERRRGKGHDGLYQGLSTATKDTYDALHMQALPPRVDGSGATNFSLL KQAGDVEENPGPMDYPTLLLALLHVYRALCEEVLWHTSVPFAENMSL ECVYPSMGILTQVEWFKIGTQQDSIAIFSPTHGMVIRKPYAERVYFLNS TMASNNMTLFFRNASEDDVGYYSCSLYTYPQGTWQKVIQVVQSDSFE AAVPSNSHIVSEPGKNVTLTCQPQMTWPVQAVRWEKIQPRQIDLLTYC NLVHGRNFTSKFPRQIVSNCSHGRWSVIVIPDVTVSDSGLYRCYLQASA GENETFVMRLTVAEGKTDNQYTLFVATRFWVLVVVGGVLACYSLLVT VAFIIFWVRSKRSRGGHSDYMNMTPRRPGPTRKHYQPYAPPRDFAAYR S* |
| 13 | 8h | TTTPAPRPPTPAPTIASQPLSLRPEACRPAAGGAVHTRGLDFACD |
| 14 | 8TM | IYIWAPLAGTCGVLLLSLVIT |
| 15 | 4-1BB | LYCKRGRKKLLYIFKQPFMRPVQTTQEEDGCSCRFPEEEEGGCEL |
| 16 | CD3ζ | RVKFSRSADAPAYKQGQNQLYNELNLGRREEYDVLDKRRGRDPEMG GKPRRKNPQEGLYNELQKDKMAEAYSEIGMKGERRRGKGHDGLYQG LSTATKDTYDALHMQALPPR |
| 17 | 28TM | FWVLVVVGGVLACYSLLVTVAFIIFWV |
| 18 | CD28 ICD | RSKRSRGGHSDYMNMTPRRPGPTRKHYQPYAPPRDFAAYRS |
| 19 | G4h | ESKYGPPCPPCPAPEFLGGPSVFLFPPKPKDTLMISRTPEVTCVVVDVSQ EDPEVQFNWYVDGVEVHNAKTKPREEQFNSTYRVVSVLTVLHQDWL NGKEYKCKVSNKGLPSSIEKTISKAKGQPREPQVYTLPPSQEEMTKNQ VSLTCLVKGFYPSDIAVEWESNGQPENNYKTTPPVLDSDGSFFLYSRLT VDKSRWQEGNVFSCSVMHEALHNHYTQKSLSLSLGK |
| 20 | CD134 ICD | ALYLLRRDQRLPPDAHKPPGGGSFRTPIQEEQADAHSTLAKI |
| 21 | 7h | APPRASALPAPPTGSALPDPQTASALPDPPAASALP |
| 22 | 8h(dc) | KPTTTPAPRPPTPAPTIASQPLSLRPEARPAAGGAVHTRGLDFAD |
| 23 | Signal peptide used in CAR | MALPVTALLLPLALLLHAARP |
| 24 | P2A | GSGATNFSLLKQAGDVEENPGP |
| 25 | CD226 nucleotide sequence | atggattatcctactttacttttggctcttcttcatgtatacagagctctatgtgaagaggtgctttggcatacatcagtt ccctttgccgagaacatgtctctagaatgtgtgtatccatcaatgggcatcttaacacaggtggagtggttcaagat cgggacccagcaggattccatagccattttcagccctactcatggcatggtcataaggaagccctatgctgagag ggtttactttttgaactcaacgatggcttccaataacatgactcttttctttcggaatgcctctgaagatgatgttggcta ctattcctgctctctttacacttacccacagggaacttggcagaaggtgatacaggtggttcagtcagatagttttga ggcagctgtgccatcaaatagccacattgtttcggaacctggaaagaatgtcacactcacttgtcagcctcagatg acgtggcctgtgcaggcagtgaggtgggaaaagatccagccccgtcagatcgacctcttaacttactgcaacttg gtccatggcagaaatttcacctccaagttcccaagacaaatagtgagcaactgcagccacggaaggtggagcgt catcgtcatccccgatgtcacagtctcagactcggggctttaccgctgctacttgcaggccagcgcaggagaaaa cgaaaccttcgtgatgagattgactgtagccgagggtaaaaccgataaccaatataccctctttgtggct |
| 26 | CD226 fusion protein nucleotide sequence | atggattatcctactttacttttggctcttcttcatgtatacagagctctatgtgaagaggtgctttggcatacatcagtt ccctttgccgagaacatgtctctagaatgtgtgtatccatcaatgggcatcttaacacaggtggagtggttcaagat cgggacccagcaggattccatagccattttcagccctactcatggcatggtcataaggaagccctatgctgagag ggtttactttttgaactcaacgatggcttccaataacatgactcttttctttcggaatgcctctgaagatgatgttggcta ctattcctgctctctttacacttacccacagggaacttggcagaaggtgatacaggtggttcagtcagatagttttga ggcagctgtgccatcaaatagccacattgtttcggaacctggaaagaatgtcacactcacttgtcagcctcagatg acgtggcctgtgcaggcagtgaggtgggaaaagatccagccccgtcagatcgacctcttaacttactgcaacttg gtccatggcagaaatttcacctccaagttcccaagacaaatagtgagcaactgcagccacggaaggtggagcgt catcgtcatccccgatgtcacagtctcagactcggggctttaccgctgctacttgcaggccagcgcaggagaaaa cgaaaccttcgtgatgagattgactgtagccgagggtaaaaccgataaccaatataccctctttgtggctactagat |
| | | tctgggtgctggtcgtggtgggtggcgtgctggcctgctacagcctgctggtgacagtggccttcatcatcttttgg gtgaggagcaagcggagcagaggcggccacagcgactacatgaacatgactccccgccgccccgggccca cccgcaagcattaccagccctatgccccaccacgcgacttcgccgcctaccggagc |
| 27 | Hypoxia promoter | atcccacagtgcatacgtgggctccaacaggtcctcttgtcgagccacagtgcatacgtgggctccaacaggtcc tcttgtcgagccacagtgcatacgtgggctccaacaggtcctcttgtcgagccacagtgcatacgtgggctccaa caggtcctcttgtcgagccacagtgcatacgtgggctccaacaggtcctcttgtcgagatctggtaggcgtgtacg gtgggaggtctatataagcagagctcgtttagtgaaccgtcagatcactag |
| 28 | Normoxia promoter | ggatctgcgatcgctccggtgcccgtcagtgggcagagcgcacatcgcccacagtccccgagaagttggggg gaggggtcggcaattgaacgggtgcctagagaaggtggcgcggggtaaactgggaaagtgatgtcgtgtactg gctccgcctttttcccgagggtgggggagaaccgtatataagtgcagtagtcgccgtgaacgttctttttcgcaac gggtttgccgccagaacacagctgaagcttcgaggggctcgcatctctccttcacgcgcccgccgccctacctg aggccgccatccacgccggttgagtcgcgttctgccgcctcccgcctgtggtgcctcctgaactgcgtccgccgt ctaggtaagtttaaagctcaggtcgagaccgggcctttgtccggcgctcccttggagcctacctagactcagccg gctctccacgctttgcctgaccctgcttgctcaactctacgtctttgtttcgttttctgttctgcgccgttacagatccaa gctgtgaccggcgcctact |
| 29 | CEA CAR nucleotide sequence | gacatccagctgacccagtcccccagctccctgtccgcctctgtgggcgacagggtgaccatcacatgcaagg cctctcaggatgtgggcacaagcgtggcctggtatcagcagaagcccggcaaggcccctaagctgctgatctat tggacctccacacggcacaccggcgtgcccagccggttcagcggctccggctctggcacagacttcaccttca ccatcagcagcctccagcccgaggacatcgccacctactattgtcagcagtacagcctgtatcggtcctttggcc agggcacaaaggtggagatcaagggatccacttccggttcaggaaagcccgggagtggtgaaggtagcacta aaggcgaggtgcagctggtggagagcggaggaggagtggtgcagcctggccggtccctgagactgtcctgct ctgccagcggcttcgactttaccacatactggatgtcctgggtgcggcaggcaccaggcaagggcctggagtg gatcggcgagatccaccccgatagctccaccatcaactatgccccttccctgaaggacaggttcaccatctctcg cgataacgccaagaatacactgttcctccagatggactctctgaggcccgaggatacaggcgtgtacttctgtgc cagcctgtatttcggctttccatggtttgcatactggggacagggcaccccagtgacagtgtctagcctcgagacc acgacgccagcgccgcgaccaccaacaccggcgcccaccatcgcgtcgcagcccctgtccctgcgcccaga ggcgtgccggccagcggcggggggcgcagtgcacacgagggggctggacttcgcctgtgatatctacatctg ggcgcccttggccgggacttgtggggtccttctcctgtcactggttatcaccctttactgcaaacggggcagaaag aaactcctgtatatattcaaacaaccatttatgagaccagtacaaactactcaagaggaagatggctgtagctgcc gatttccagaagaagaagaaggaggatgtgaactgagagtgaagttcagcaggagcgcagacgcccccgcgt acaagcagggccagaaccagctctataacgagctcaatctaggacgaagagaggagtacgatgttttggacaa gagacgtggccgggaccctgagatggggggaaagccgagaaggaagaaccctcaggaaggcctgtacaat gaactgcagaaagataagatggcggaggcctacagtgagattgggatgaaaggcgagcgccggaggggcaa ggggcacgatggcctttaccagggtctcagtacagccaccaaggacacctacgacgcccttcacatgcaggcc ctgccccctcgc |
| 30 | CEA CD226 CAR nucleotide | gacatccagctgacccagtcccccagctccctgtccgcctctgtgggcgacagggtgaccatcacatgcaagg cctctcaggatgtgggcacaagcgtggcctggtatcagcagaagcccggcaaggcccctaagctgctgatctat tggacctccacacggcacaccggcgtgcccagccggttcagcggctccggctctggcacagacttcaccttca |
| | sequence | ccatcagcagcctccagcccgaggacatcgccacctactattgtcagcagtacagcctgtatcggtcctttggcc agggcacaaaggtggagatcaagggatccacttccggttcaggaaagcccgggagtggtgaaggtagcacta aaggcgaggtgcagctggtggagagcggaggaggagtggtgcagcctggccggtccctgagactgtcctgct ctgccagcggcttcgactttaccacatactggatgtcctgggtgcggcaggcaccaggcaagggcctggagtg gatcggcgagatccaccccgatagctccaccatcaactatgccccttccctgaaggacaggttcaccatctctcg cgataacgccaagaatacactgttcctccagatggactctctgaggcccgaggatacaggcgtgtacttctgtgc cagcctgtatttcggctttccatggtttgcatactggggacagggcaccccagtgacagtgtctagcctcgagacc acgacgccagcgccgcgaccaccaacaccggcgcccaccatcgcgtcgcagcccctgtccctgcgcccaga ggcgtgccggccagcggcggggggcgcagtgcacacgagggggctggacttcgcctgtgatatctacatctg ggcgcccttggccgggacttgtggggtccttctcctgtcactggttatcaccctttactgcaaacggggcagaaag aaactcctgtatatattcaaacaaccatttatgagaccagtacaaactactcaagaggaagatggctgtagctgcc gatttccagaagaagaagaaggaggatgtgaactgagagtgaagttcagcaggagcgcagacgcccccgcgt acaagcagggccagaaccagctctataacgagctcaatctaggacgaagagaggagtacgatgttttggacaa gagacgtggccgggaccctgagatggggggaaagccgagaaggaagaaccctcaggaaggcctgtacaat gaactgcagaaagataagatggcggaggcctacagtgagattgggatgaaaggcgagcgccggaggggcaa ggggcacgatggcctttaccagggtctcagtacagccaccaaggacacctacgacgcccttcacatgcaggcc ctgccccctcgcgtcgacggaagcggagctactaacttcagcctgctgaagcaggctggagacgtggaggag aaccctggacctatggattatcctactttacttttggctcttcttcatgtatacagagctctatgtgaagaggtgctttg gcatacatcagttccctttgccgagaacatgtctctagaatgtgtgtatccatcaatgggcatcttaacacaggtgg agtggttcaagatcgggacccagcaggattccatagccattttcagccctactcatggcatggtcataaggaagc cctatgctgagagggtttactttttgaactcaacgatggcttccaataacatgactcttttctttcggaatgcctctgaa gatgatgttggctactattcctgctctctttacacttacccacagggaacttggcagaaggtgatacaggtggttca gtcagatagttttgaggcagctgtgccatcaaatagccacattgtttcggaacctggaaagaatgtcacactcactt gtcagcctcagatgacgtggcctgtgcaggcagtgaggtgggaaaagatccagccccgtcagatcgacctctta acttactgcaacttggtccatggcagaaatttcacctccaagttcccaagacaaatagtgagcaactgcagccacg gaaggtggagcgtcatcgtcatccccgatgtcacagtctcagactcggggctttaccgctgctacttgcaggcca gcgcaggagaaaacgaaaccttcgtgatgagattgactgtagccgagggtaaaaccgataaccaatataccctc tttgtggctactagattctgggtgctggtcgtggtgggtggcgtgctggcctgctacagcctgctggtgacagtgg ccttcatcatcttttgggtgaggagcaagcggagcagaggcggccacagcgactacatgaacatgactccccgc cgccccgggcccacccgcaagcattaccagccctatgccccaccacgcgacttcgccgcctaccggagctaa |
| 31 | PSCA CAR nucleotide sequence | gacatcgagatgacccagtctccatcctccctgtctgcatctgtaggagacagagtcaccatcacttgccgggca agtcagagcatttccagccatttaaattggtatcagcagaaatcagggaaagcccctaagctcctgatctatgctg cgtccagtttgcaaggtggggtcccatcaaggttcagtggcagtggatctgggacagatttcactctcaccatcag cagtctgcaacctgaagattttgcaacttactactgtcaacagagttacagtcccccgtggacgttcggccaaggg accaagctggaactcaaacgtggcagcacaagcggaagcggcaaaccaggaagcggagaaggaagcacca agggagaagtgcagctggtgcagtctggggctgaggtgaagaagcctgggtcctcggtgaaggtctcctgcaa ggcttctggaggcaccttcagcagctatgctatcagctgggtgcgacaggcccctggacaagggcttgagtgga tgggagggatcatccctatctttggtacagcaaactacgcacagaagttccagggcagagtcacgattaccgcg |
| | | gacgaatccacgagcacagcctacatggagctgagcagcctgagatctgaggacacggccgtgtattactgtg cgagagataccatgatagtagcaaatgcttttgatatctggggccaagggacaacggtcaccgtctcctcgctcg agaccacgacgccagcgccgcgaccaccaacaccggcgcccaccatcgcgtcgcagcccctgtccctgcgc ccagaggcgtgccggccagcggcggggggcgcagtgcacacgagggggctggacttcgcctgtgatatcta catctgggcgcccttggccgggacttgtggggtccttctcctgtcactggttatcaccctttactgcaaacggggc agaaagaaactcctgtatatattcaaacaaccatttatgagaccagtacaaactactcaagaggaagatggctgta gctgccgatttccagaagaagaagaaggaggatgtgaactgagagtgaagttcagcaggagcgcagacgccc ccgcgtacaagcagggccagaaccagctctataacgagctcaatctaggacgaagagaggagtacgatgttttg gacaagagacgtggccgggaccctgagatggggggaaagccgagaaggaagaaccctcaggaaggcctgt acaatgaactgcagaaagataagatggcggaggcctacagtgagattgggatgaaaggcgagcgccggagg ggcaaggggcacgatggcctttaccagggtctcagtacagccaccaaggacacctacgacgcccttcacatgc aggccctgccccctcgctaa |
| 32 | PSCA CD226 CAR nucleotide sequence | gacatcgagatgacccagtctccatcctccctgtctgcatctgtaggagacagagtcaccatcacttgccgggca agtcagagcatttccagccatttaaattggtatcagcagaaatcagggaaagcccctaagctcctgatctatgctg cgtccagtttgcaaggtggggtcccatcaaggttcagtggcagtggatctgggacagatttcactctcaccatcag cagtctgcaacctgaagattttgcaacttactactgtcaacagagttacagtcccccgtggacgttcggccaaggg accaagctggaactcaaacgtggcagcacaagcggaagcggcaaaccaggaagcggagaaggaagcacca agggagaagtgcagctggtgcagtctggggctgaggtgaagaagcctgggtcctcggtgaaggtctcctgcaa ggcttctggaggcaccttcagcagctatgctatcagctgggtgcgacaggcccctggacaagggcttgagtgga tgggagggatcatccctatctttggtacagcaaactacgcacagaagttccagggcagagtcacgattaccgcg gacgaatccacgagcacagcctacatggagctgagcagcctgagatctgaggacacggccgtgtattactgtg cgagagataccatgatagtagcaaatgcttttgatatctggggccaagggacaacggtcaccgtctcctcgctcg agaccacgacgccagcgccgcgaccaccaacaccggcgcccaccatcgcgtcgcagcccctgtccctgcgc ccagaggcgtgccggccagcggcggggggcgcagtgcacacgagggggctggacttcgcctgtgatatcta catctgggcgcccttggccgggacttgtggggtccttctcctgtcactggttatcaccctttactgcaaacggggc agaaagaaactcctgtatatattcaaacaaccatttatgagaccagtacaaactactcaagaggaagatggctgta gctgccgatttccagaagaagaagaaggaggatgtgaactgagagtgaagttcagcaggagcgcagacgccc ccgcgtacaagcagggccagaaccagctctataacgagctcaatctaggacgaagagaggagtacgatgttttg gacaagagacgtggccgggaccctgagatggggggaaagccgagaaggaagaaccctcaggaaggcctgt acaatgaactgcagaaagataagatggcggaggcctacagtgagattgggatgaaaggcgagcgccggagg ggcaaggggcacgatggcctttaccagggtctcagtacagccaccaaggacacctacgacgcccttcacatgc aggccctgccccctcgcgtcgacggaagcggagctactaacttcagcctgctgaagcaggctggagacgtgg aggagaaccctggacctatggattatcctactttacttttggctcttcttcatgtatacagagctctatgtgaagaggt gctttggcatacatcagttccctttgccgagaacatgtctctagaatgtgtgtatccatcaatgggcatcttaacaca ggtggagtggttcaagatcgggacccagcaggattccatagccattttcagccctactcatggcatggtcataag gaagccctatgctgagagggtttactttttgaactcaacgatggcttccaataacatgactcttttctttcggaatgcc tctgaagatgatgttggctactattcctgctctctttacacttacccacagggaacttggcagaaggtgatacaggtg gttcagtcagatagttttgaggcagctgtgccatcaaatagccacattgtttcggaacctggaaagaatgtcacact |
| | | cacttgtcagcctcagatgacgtggcctgtgcaggcagtgaggtgggaaaagatccagccccgtcagatcgac ctcttaacttactgcaacttggtccatggcagaaatttcacctccaagttcccaagacaaatagtgagcaactgcag ccacggaaggtggagcgtcatcgtcatccccgatgtcacagtctcagactcggggctttaccgctgctacttgca ggccagcgcaggagaaaacgaaaccttcgtgatgagattgactgtagccgagggtaaaaccgataaccaatat accctctttgtggctactagattctgggtgctggtcgtggtgggtggcgtgctggcctgctacagcctgctggtga cagtggccttcatcatcttttgggtgaggagcaagcggagcagaggcggccacagcgactacatgaacatgact ccccgccgccccgggcccacccgcaagcattaccagccctatgccccaccacgcgacttcgccgcctaccgg agctaa |
| 33 | CD70 CAR nucleotide sequence | gacatccagatgacccagtctccatcctccctgtctgcatctgtaggagacagagtcaccatcacttgccgggca agtcagagcattagcagctatttaaattggtatcagcagaaaccagggaaagcccctaagctcctgatctttgctg catccagtttgcaaagtggggtcccatcaaggttcagtggcagtggatctgggacagatttcactctcaccatcag cagtctgcaacctgaagattttgcaacttactactgtcaacagagttacagtaccccctggacgttcggccaaggg accaaactggatatcaaacgtggcagcacaagcggaagcggcaaaccaggaagcggagaaggaagcacca agggagaggtgcagctggtgcagtccggaccagaggtgaagaagccaggagccagcgtgaaggtgtcctgt aaggcctctggctacaccttcacagattactatatgaactgggtgcggcagatgcacggcaagggactggagtg gatgggcgtgatcaacccatacaatggcggcaccgattataatcagaagtttaagggcagagtgaccatcacag ccgacaagtccacctctacagcctacatggagctgagctccctgaggagcgaggacacagccgtgtactattgt gcccgctccgtgtacgactatccctttgattattggggccagggcaccctggtcacagtctcctcactcgagacca cgacgccagcgccgcgaccaccaacaccggcgcccaccatcgcgtcgcagcccctgtccctgcgcccagag gcgtgccggccagcggcggggggcgcagtgcacacgagggggctggacttcgcctgtgatatctacatctgg gcgcccttggccgggacttgtggggtccttctcctgtcactggttatcaccctttactgcaaacggggcagaaaga aactcctgtatatattcaaacaaccatttatgagaccagtacaaactactcaagaggaagatggctgtagctgccg atttccagaagaagaagaaggaggatgtgaactgagagtgaagttcagcaggagcgcagacgcccccgcgta caagcagggccagaaccagctctataacgagctcaatctaggacgaagagaggagtacgatgttttggacaag agacgtggccgggaccctgagatggggggaaagccgagaaggaagaaccctcaggaaggcctgtacaatga actgcagaaagataagatggcggaggcctacagtgagattgggatgaaaggcgagcgccggaggggcaagg ggcacgatggcctttaccagggtctcagtacagccaccaaggacacctacgacgcccttcacatgcaggccctg ccccctcgctaa |
| 34 | CD70 CD226CAR nucleotide sequence | gacatccagatgacccagtctccatcctccctgtctgcatctgtaggagacagagtcaccatcacttgccgggca agtcagagcattagcagctatttaaattggtatcagcagaaaccagggaaagcccctaagctcctgatctttgctg catccagtttgcaaagtggggtcccatcaaggttcagtggcagtggatctgggacagatttcactctcaccatcag cagtctgcaacctgaagattttgcaacttactactgtcaacagagttacagtaccccctggacgttcggccaaggg accaaactggatatcaaacgtggcagcacaagcggaagcggcaaaccaggaagcggagaaggaagcacca agggagaggtgcagctggtgcagtccggaccagaggtgaagaagccaggagccagcgtgaaggtgtcctgt aaggcctctggctacaccttcacagattactatatgaactgggtgcggcagatgcacggcaagggactggagtg gatgggcgtgatcaacccatacaatggcggcaccgattataatcagaagtttaagggcagagtgaccatcacag ccgacaagtccacctctacagcctacatggagctgagctccctgaggagcgaggacacagccgtgtactattgt gcccgctccgtgtacgactatccctttgattattggggccagggcaccctggtcacagtctcctcactcgagacca |
| | | cgacgccagcgccgcgaccaccaacaccggcgcccaccatcgcgtcgcagcccctgtccctgcgcccagag gcgtgccggccagcggcggggggcgcagtgcacacgagggggctggacttcgcctgtgatatctacatctgg gcgcccttggccgggacttgtggggtccttctcctgtcactggttatcaccctttactgcaaacggggcagaaaga aactcctgtatatattcaaacaaccatttatgagaccagtacaaactactcaagaggaagatggctgtagctgccg atttccagaagaagaagaaggaggatgtgaactgagagtgaagttcagcaggagcgcagacgcccccgcgta caagcagggccagaaccagctctataacgagctcaatctaggacgaagagaggagtacgatgttttggacaag agacgtggccgggaccctgagatggggggaaagccgagaaggaagaaccctcaggaaggcctgtacaatga actgcagaaagataagatggcggaggcctacagtgagattgggatgaaaggcgagcgccggaggggcaagg ggcacgatggcctttaccagggtctcagtacagccaccaaggacacctacgacgcccttcacatgcaggccctg ccccctcgcgtcgacggaagcggagctactaacttcagcctgctgaagcaggctggagacgtggaggagaac cctggacctatggattatcctactttacttttggctcttcttcatgtatacagagctctatgtgaagaggtgctttggcat acatcagttccctttgccgagaacatgtctctagaatgtgtgtatccatcaatgggcatcttaacacaggtggagtg gttcaagatcgggacccagcaggattccatagccattttcagccctactcatggcatggtcataaggaagccctat gctgagagggtttactttttgaactcaacgatggcttccaataacatgactcttttctttcggaatgcctctgaagatg atgttggctactattcctgctctctttacacttacccacagggaacttggcagaaggtgatacaggtggttcagtcag atagttttgaggcagctgtgccatcaaatagccacattgtttcggaacctggaaagaatgtcacactcacttgtcag cctcagatgacgtggcctgtgcaggcagtgaggtgggaaaagatccagccccgtcagatcgacctcttaactta ctgcaacttggtccatggcagaaatttcacctccaagttcccaagacaaatagtgagcaactgcagccacggaag gtggagcgtcatcgtcatccccgatgtcacagtctcagactcggggctttaccgctgctacttgcaggccagcgc aggagaaaacgaaaccttcgtgatgagattgactgtagccgagggtaaaaccgataaccaatataccctctttgt ggctactagattctgggtgctggtcgtggtgggtggcgtgctggcctgctacagcctgctggtgacagtggcctt catcatcttttgggtgaggagcaagcggagcagaggcggccacagcgactacatgaacatgactccccgccgc cccgggcccacccgcaagcattaccagccctatgccccaccacgcgacttcgccgcctaccggagctaa |
| 35 | CD70 scFv | DIQMTQSPSSLSASVGDRVTITCRASQSISSYLNWYQQKPGKAPKLLIFA ASSLQSGVPSRFSGSGSGTDFTLTISSLQPEDFATYYCQQSYSTPWTFGQ GTKLDIKRGSTSGSGKPGSGEGSTKGEVQLVQSGPEVKKPGASVKVSC KASGYTFTDYYMNWVRQMHGKGLEWMGVINPYNGGTDYNQKFKGR VTITADKSTSTAYMELSSLRSEDTAVYYCARSVYDYPFDYWGQGTLVT VSS |

## Claims

1. A fusion protein, from N-terminus to C-terminus, comprising or consisting of:
1) a CD226 extracellular region, a transmembrane domain, and a co-stimulatory domain; or
2) a signal peptide, a CD226 extracellular region, a transmembrane domain, and a co-stimulatory domain.

2. The fusion protein according to claim 1, wherein the transmembrane domain comprises or consists of a transmembrane domain of any one or more molecules selected from the group consisting of: ICOS, CD4, CD8α, CD28, CD3ζ, and CD226.

3. The fusion protein according to claim 1 or 2, wherein the co-stimulatory domain comprises or consists of a signal transduction domain of any one or more molecules selected from the group consisting of:
CD27, CD28, 4-1BB, OX40, CD30, CD40, CD2, LFA-1, LIGHT, NKG2C, B7-H3, PD-1, ICOS, CDS, ICAM-1, GITR, BAFFR, LIGHTR, SLAMF7, CD7, NKp80 (KLRF1), CD160, CD19, CD4, CD8α, CD8β, IL2Rβ, IL2Rγ, IL7Rα, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, TNFR2, TRANCE/RANKL, DNAMl, SLAMF4, CD84, CD96, CEACAM1, CRTAM, CD229, CD160, PSGL1, CD100, CD69, SLAMF6, SLAMF1, SLAMF8, CD162, LTBR, LAT, GADS, SLP-76, PAG/Cbp, NKp44, NKp30, NKp46, NKG2D, and a ligand specifically binding to CD83.

4. The fusion protein according to any one of claims 1 to 3, wherein the signal peptide comprises or is a signal peptide of a CD226 or CD8α molecule.

5. The fusion protein according to any one of claims 1 to 4, from the N-terminus to the C-terminus, comprising or consisting of:
1) the CD226 extracellular region, a CD28 transmembrane domain, and a CD28 signal transduction domain; or
2) a CD226 signal peptide, the CD226 extracellular region, a CD28 transmembrane domain, and a CD28 signal transduction domain.

6. The fusion protein according to any one of claims 1 to 5, wherein the CD226 extracellular region comprises or consists of an amino acid sequence as set forth in SEQ ID NO:1, or an amino acid sequence having at least 85% sequence identity to SEQ ID NO:1.

7. The fusion protein according to claim 6, comprising or consisting of an amino acid sequence as set forth in SEQ ID NO:2, or an amino acid sequence having at least 85% sequence identity to SEQ ID NO:2.

8. An engineered receptor, comprising the fusion protein according to any one of claims 1 to 6, and a primary signal transduction domain located at the C-terminus of the fusion protein.

9. The engineered receptor according to claim 8, wherein the primary signal transduction domain comprises or consists of a signal transduction domain of one or more molecules selected from the group consisting of: CD3ζ, CD3γ, CD3δ, CD3ε, CD5, CD22, FcRy, FcRβ, FcεRIγ, FcεRIβ, FcγRIIa, CD79α, CD79β, CD66d, DAP10, and DAP12.

10. An engineered nucleic acid molecule, encoding the fusion protein according to any one of claims 1 to 7 or the engineered receptor according to claim 8 or 9.

11. An engineered cell, comprising the fusion protein according to any one of claims 1 to 7 or the engineered receptor according to claim 8 or 9 and/or the engineered nucleic acid molecule according to claim 10.

12. The engineered cell according to claim 11, further comprising one or more engineered receptors binding to one or more target molecules, wherein
the one or more target molecules are one or more selected from CD70, PSCA, and CEA;
the engineered receptor is one or more selected from CAR, TCR, and TAC.

13. The engineered cell according to claim 12, wherein the engineered receptor binding to other molecules is a CEA-binding CAR.

14. The engineered cell according to claim 13, wherein the CEA-binding CAR comprises or consists of an amino acid sequence as set forth in SEQ ID NO:6 or a conservative substitution variant thereof, or an amino acid sequence having at least 85% sequence identity thereto.

15. The engineered cell according to claim 12, wherein the engineered receptor binding to other molecules is a PSCA-binding CAR.

16. The engineered cell according to claim 15, wherein the PSCA-binding CAR comprises or consists of an amino acid sequence as set forth in SEQ ID NO:9 or a conservative substitution variant thereof, or an amino acid sequence having at least 85% sequence identity thereto.

17. The engineered cell according to claim 12, wherein the engineered receptor binding to other molecules is a CD70-binding CAR.

18. The engineered cell according to claim 17, wherein the CD70-binding CAR comprises or consists of an amino acid sequence as set forth in SEQ ID NO: 12 or a conservative substitution variant thereof, or an amino acid sequence having at least 85% sequence identity thereto.

19. The engineered cell according to any one of claims 11 to 18, which is derived from a T cell, NK cell, macrophage, DC cell, B cell, or a precursor cell thereof.

20. Use of the fusion protein according to any one of claims 1 to 7, the engineered receptor according to claim 8 or 9, the engineered nucleic acid molecule according to claim 10, the engineered cell according to any one of claims 11 to 19 in manufacture of a medicament for treating cancer.

21. The use according to claim 20, wherein the cancer is one or more selected from the group consisting of: bladder cancer, blood cancer, bone cancer, bone marrow cancer, brain/nervous system cancer, breast cancer, colorectal cancer, esophageal cancer, gastrointestinal cancer, head cancer, kidney cancer, liver cancer, lung cancer, nasopharyngeal cancer, neck cancer, ovarian cancer, pancreatic cancer, prostate cancer, skin cancer, gastric cancer, testicular cancer, tongue cancer, and uterine cancer.

22. A method for prolonging an *in vivo* persistence period of CAR-T cells, comprising expressing the fusion protein according to any one of claims 1 to 7 or the engineered receptor according to claim 8 or 9 on a membrane of the CAR-T cells.

23. A method for enhancing an *in vivo* expansion ability of CAR-T cells, comprising expressing the fusion protein according to any one of claims 1 to 7 or the engineered receptor according to claim 8 or 9 on a membrane of the CAR-T cells.

24. A method for enhancing an *in vivo* killing ability of CAR-T cells against target cells, comprising expressing the fusion protein according to any one of claims 1 to 7 or the engineered receptor according to claim 8 or 9 on a membrane of the CAR-T cells.
